(19) Europäisches Patentamt
European Patent Office
Office européen des brevets

(11) EP 0 535 635 B1

(12) **EUROPEAN PATENT SPECIFICATION**

(45) Date of publication and mention of the grant of the patent:
**21.08.1996 Bulletin 1996/34**

(51) Int. Cl.[6]: **C07C 255/50**, C09K 19/18, C07C 25/24, G02F 1/13

(21) Application number: **92116733.4**

(22) Date of filing: **30.09.1992**

(54) **Tolane derivative, liquid crystal composition containing the derivative, and liquid crystal display device using the composition**

Tolanderivate, sie enthaltende flüssigkristalline Zusammensetzungen und deren Verwendung in Flüssigkristalline-Anzeigeelementen

Dérivés du tolane, compositions de cristaux liquides les contenant, et dispositifs d'affichage à cristaux liquides utilisant lesdites compositions

(84) Designated Contracting States:
**CH DE GB LI**

(30) Priority: **02.10.1991 JP 255455/91**
**02.12.1991 JP 318063/91**
**06.03.1992 JP 49894/92**
**13.05.1992 JP 120735/92**
**25.05.1992 JP 131246/92**
**29.06.1992 JP 170328/92**

(43) Date of publication of application:
**07.04.1993 Bulletin 1993/14**

(73) Proprietor: **SEIKO EPSON CORPORATION**
**Shinjuku-ku Tokyo-to (JP)**

(72) Inventors:
- **Yamada, Shuhei,**
**c/o Seiko Epson Corporation**
**Suwa-shi, Nagano-ken (JP)**
- **Ikukawa, Shuji,**
**c/o Seiko Epson Corporation**
**Suwa-shi, Nagano-ken (JP)**
- **Nakayama, Jitsuko,**
**c/o Seiko Epson Corporation**
**Suwa-shi, Nagano-ken (JP)**

(74) Representative: **Hoffmann, Eckart, Dipl.-Ing.**
**Patentanwalt,**
**Bahnhofstrasse 103**
**82166 Gräfelfing (DE)**

(56) References cited:
**EP-A- 0 442 266** **WO-A-88/07514**

Remarks:
The file contains technical information submitted after the application was filed and not included in this specification

EP 0 535 635 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.13.2/3.4

## Description

The present invention relates to novel tolan derivatives which are used as an electroptical display material and to liquid crystal compositions containing them and liquid crystal display devices using the compositions.

Liquid crystal display devices utilize the electroptical effect of liquid crystals, and the liquid crystal phase to be used in them includes the nematic phase, the cholesteric phase and the smectic phase. The display system which is now used most widely is a twisted nematic (hereinafter referred to as TN) type system of utilizing nematic phase or a super twisted nematic (hereinafter referred to as STN) type system in which the twisted angle is enlarged.

Since liquid crystal display devices have various merits such as:

1. being small and thin,
2. having low driving voltage and small power consumption, and
3. not causing eyestrain even after use for a long period of time because of the image-receiving element;

they have heretofore been applied to watches, electronic calculators, audio instruments, various metering instruments and car dashboards. Recently, in particular, they are applied also to displays of personal computers and word processors as well as to color televisions or the like having an extremely large number of pixels, and are therefore especially noted as display devices substitutable for CRT. Thus, liquid crystal display devices are now applied to various fields, which are considered to be enlarged further in future. Along with the tendency, the necessary characteristics of liquid crystal materials are considered to vary, but the following characteristics are basic and indispensable ones.

1. The liquid crystal materials should be colorless and stable to heat and light and stable electrically and chemically.

2. They should have a broad practical temperature range.

3. They should have a rapid electroptical response speed.

4. They should need a low driving voltage only.

5. The rising of the voltage-light transmittance characteristic of them should be rapid, and the temperature dependence of the threshold voltage (Vth) of them should be small.

6. They should have a broad viewing angle range.

A lot of liquid crystals are known which satisfy the characteristic 1 but no liquid crystal compound satisfying all the other characteristics 2 to 6 is known. In order to obtain the characteristics, liquid crystal compositions each comprising nematic liquid crystal compounds of plural kinds or containing non-liquid crystal compound(s) along with liquid crystal compound(s) are used. In order to satisfy the characteristic 2 of them, liquid crystal compounds having a low crystal nematic phase transition point (hereinafter referred to as C-N point) and having a high nematic isotropic phase transition point (hereinafter referred to as N-I point) and, as a result, having a broad nematic mesomorphic range are needed. With respect to the characteristic 3, the response speed (hereinafter referred to as $\tau$), the viscosity coefficient (hereinafter referred to as $\eta$) and the cell gap (hereinafter referred to as d) have the following relation:

$$\tau \propto \eta\, d$$

Therefore, in order to satisfy the characteristic 3, d must be small so as to elevate the response speed. In cells for practical use, since the value of $\Delta n \cdot d$ (where $\Delta n$ means refractive index anisotropy) is set to be a constant value in order to prevent generation of interference fringes on the surface of the cell, which fringes would worsen the outward appearance of the cell, the value of d may be reduced when a material having a large $\Delta n$ value is used and, as a result, the response speed may be elevated. In order to satisfy both the characteristics 2 and 3, cyclohexyltolan derivatives and phenyltolan derivatives have already been known as a liquid crystal material having a broad nematic liquid crystal range and having a large $\Delta n$ value, as described in JP-A 60-155142 and 60-152427, U.S. Patent 4,713,468 and JP-A 63-152334. (The term "JP-A" as used herein means an "unexamined published Japanese patent application.)

Table 1

| Code | Structural Formura | Transition Point | Reference |
|---|---|---|---|
| a-1 | 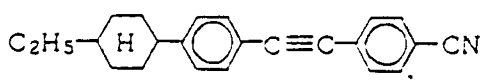 | C-N point 135°C<br>N-I point 250°C | JP-A<br>60-155142 |
| a-2 | 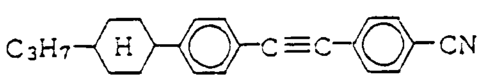 | C-N point 153°<br>N-I point 265°C | JP-A<br>60-155142 |
| b-1 | 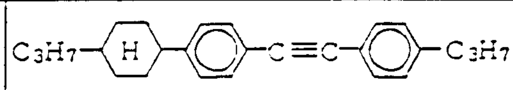 | C-N point 96°C<br>N-I point 213°C | JP-A<br>60-152427 |
| b-2 | 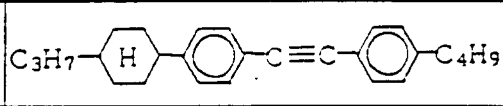 | C-N point 87°C<br>N-I point 201°C | US Patent<br>4713468 |
| c-1 | 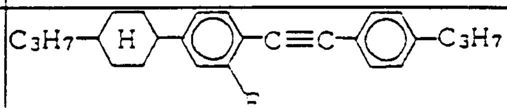 | C-N point 79°C<br>N-I point 199°C | JP-A<br>63-152334 |
| c-2 | 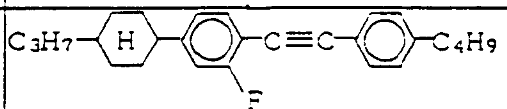 | C-N point · 50°C<br>N-I point 188°C | JP-A<br>63-152334 |
| d-1 | 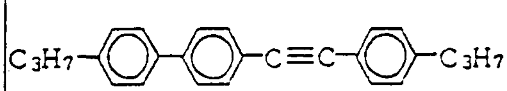 | C-N point 164°C<br>N-I point 230°C | JP-A<br>60-152427 |
| d-2 | 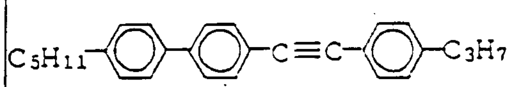 | C-N point 160°C<br>N-I point 211°C | JP-A<br>60-152427 |

Of these compounds, however, cyclohexyltolan derivatives of (a-1) to (c-2) each have a cyclohexane ring in the skeleton, which ring interferes with the effect of enlarging the value Δn. Compounds (a-1) and (a-2) each have a high C-N point and therefore are considered to have a problem on the compatibility with other liquid crystal compounds. Compounds (d-1) and (d-2), though having a large Δn value, also have an extremely high C-N point and therefore are also considered to have a problem on the compatibility.

Under this situation, the present invention is to meet the practical requirements and the object thereof is to provide novel liquid crystal compounds which may be blended with plural kinds of nematic liquid crystals or non-liquid crystals with high compatibility with them to give liquid crystal compositions having a broad practical temperature range and a large Δn value.

The present invention provides tolan derivatives of a general formula:

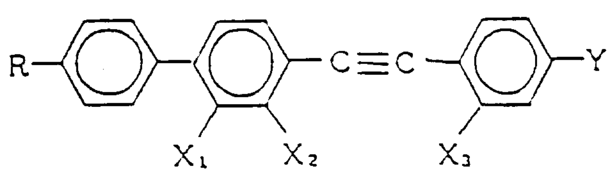 (1)

where R represents a linear alkyl group having from 1 to 10 carbon atoms; X1, X2 and X3 each represent a fluorine

atom or a hydrogen atom, provided that one of them is necessarily a fluorine atom, but X1 and X2 are not at the same time fluorine atoms, and the others are hydrogen atoms; and Y represents a nitrile group or a linear alkyl group having from 1 to 10 carbon atoms. It also provides liquid crystal compositions containing the derivative(s) and liquid crystal display devices having the composition. Compounds (1) of the present invention are obtained by anyone of the following processes.

Table 2

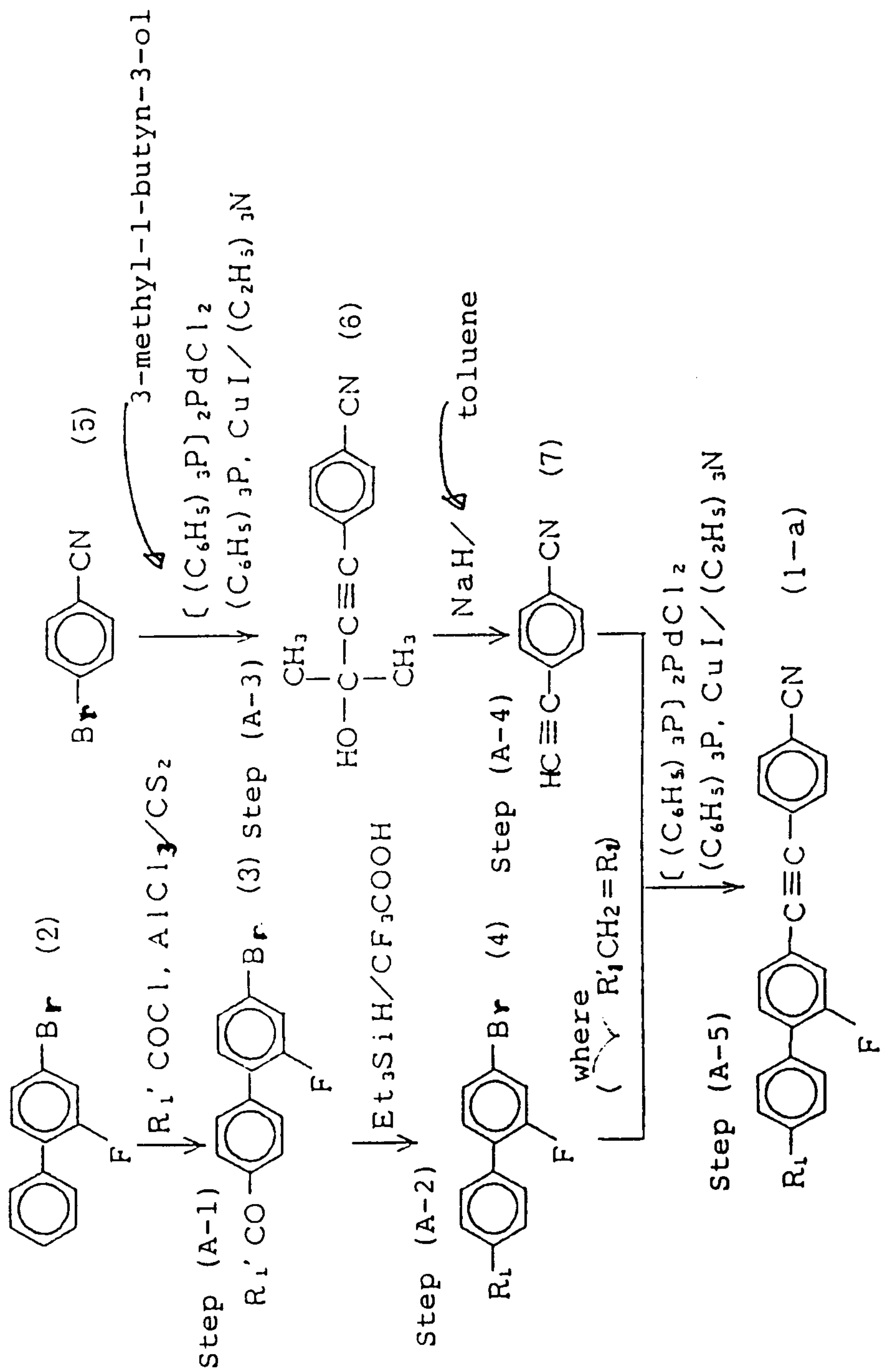

Step (A-1):

Compound (2) is reacted with an alkylcarboxylic acid chloride in carbon disulfide in the presence of aluminum chloride to obtain compound (3).

Step (A-2):

Compound (3) is reacted with triethylsilane in trifluoroacetic acid to obtain compound (4).

Step (A-3):

Compound (5) is reacted with 3-methyl-1-butyl-3-ol in triethylamine in the presence of bis(triphenylphosphine) palladium(II) chloride, triphenylphosphine and copper (I) iodide, to obtain compound (6).

Step (A-4):

Compound (6) is reacted with sodium hydride in toluene to obtain compound (7).

Step (A-5):

Compound (4) is reacted with compound (7) in triethylamine in the presence of bis(triphenylphosphine) palladium(II) chloride, triphenylphosphine and copper (I) iodide, to obtain compound (1-a).

Table 3

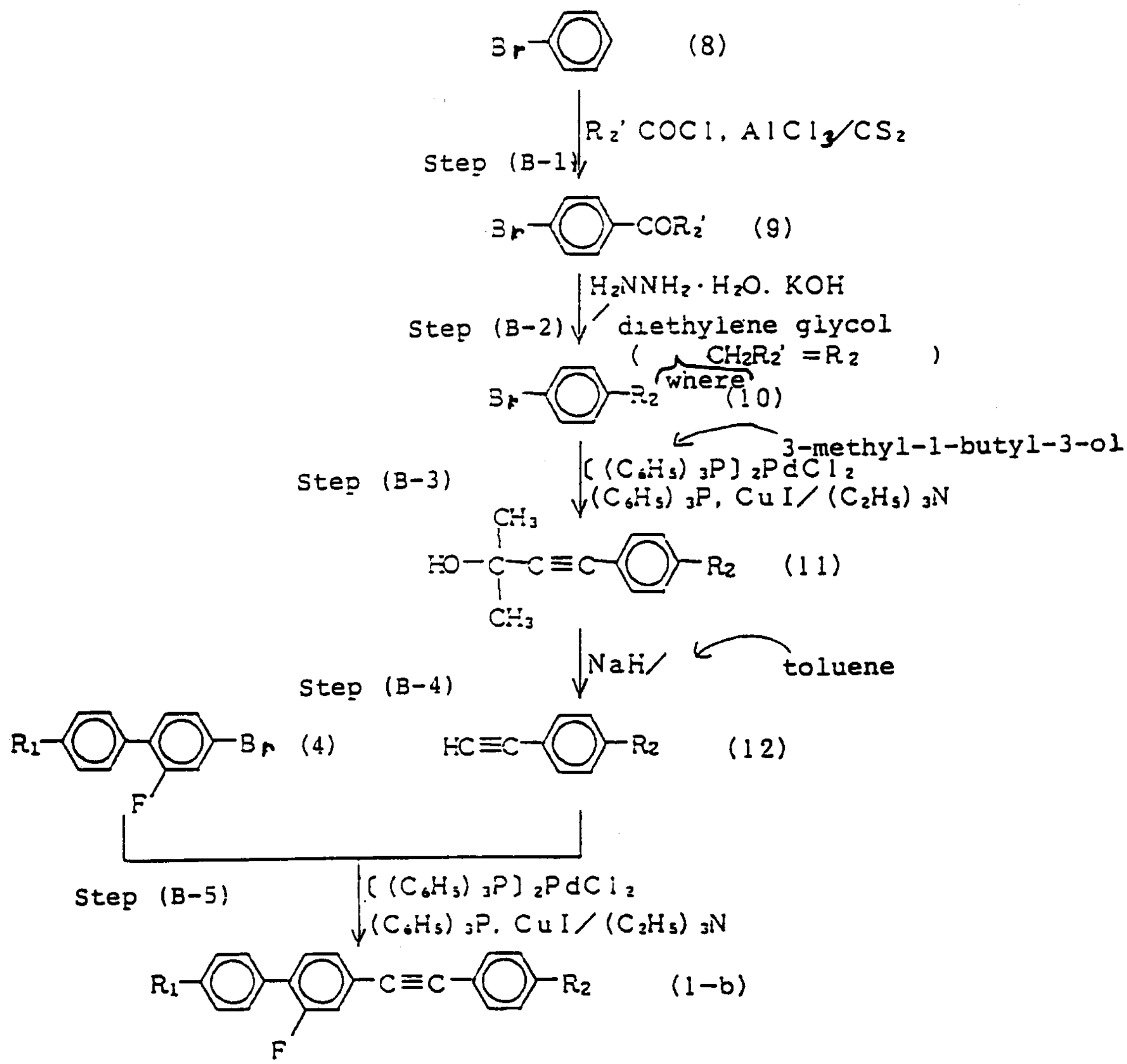


Step (B-1):

Compound (8) is reacted with an alkylcarboxylic acid chloride in carbon disulfide in the presence of aluminum chloride to obtain compound (9).

Step (B-2):

Compound (9) is reacted with hydrazine (monohydrate) in diethylene glycol in the presence of potassium hydroxide to obtain compound (10).

Step (B-3):

Compound (10) is reacted with 3-methyl-1-butyl-3-ol in triethylamine in the presence of bis(triphenylphosphine) palladium(II) chloride, triphenylphosphine and copper (I) iodide to obtain compound (11).

Step (B-4):

Compound (11) is reacted with sodium hydride in toluene to obtain compound (12).

Step (B-5):

Compound (4) is reacted with compound (12) in triethylamine in the presence of bis(triphenylphosphine) palladium(II) chloride, triphenylphosphine and copper(I) iodide to obtain compound (1-b).

**Table 4**

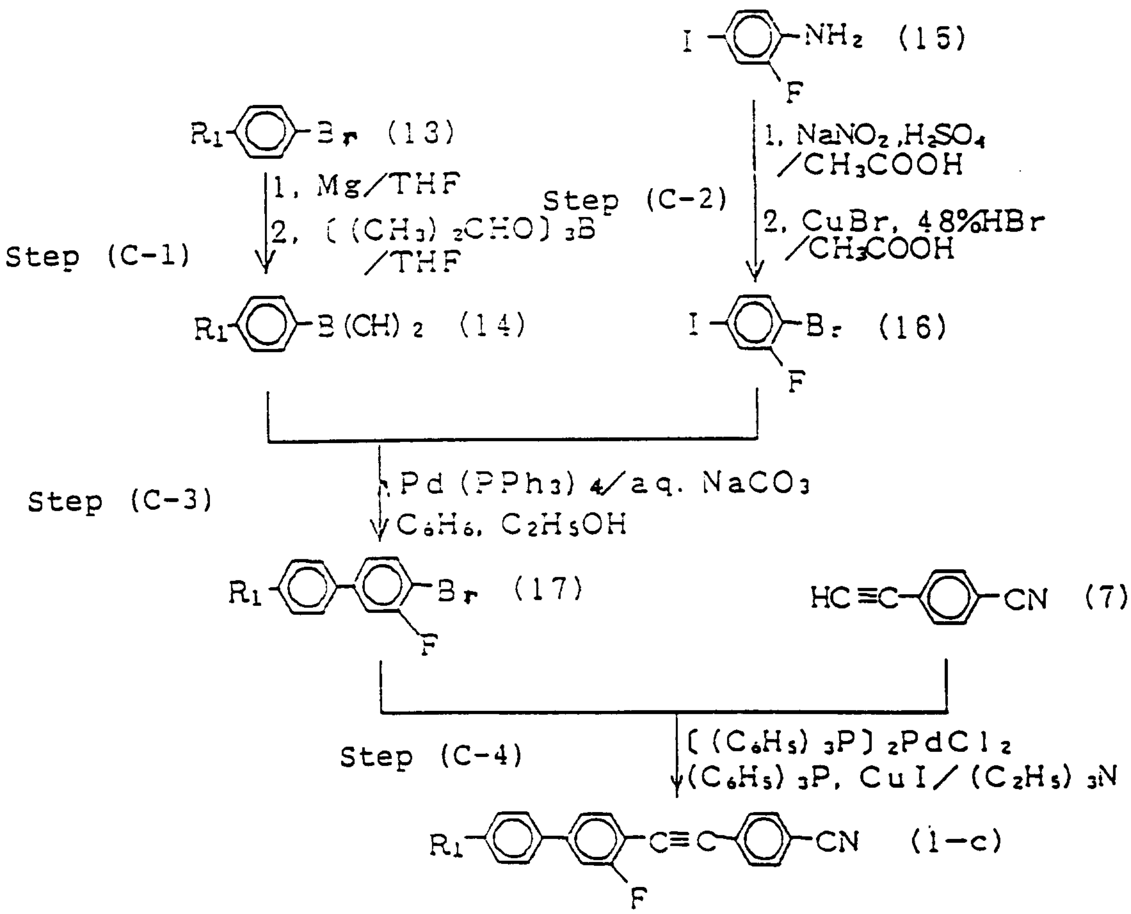


Step (C-1):

Compound (13) is formed into a Grignard reagent in tetrahydrofuran (hereinafter referred to as THF) and then reacted with trimethyl borate to obtain compound (14).

Step (C-2):

Compound (15) is reacted with sodium nitrite and sulfuric acid in acetic acid to give its diazonium salt, which is then reacted with copper(I) bromide and hydrobromic acid to obtain compound (16).

Step (C-3):

Compound (14) and compound (16) are reacted in a mixed solvent of ethanol and benzene in the presence of tetrakistriphenylphosphine palladium(0) to obtain compound (17).

Step (C-4):

Compound (17) is reacted with compound (7) in triethylamine in the presence of bis(triphenylphosphine) palladium(II) chloride, triphenylphosphine and copper(I) iodide to obtain compound (1-c).

**Table 5**

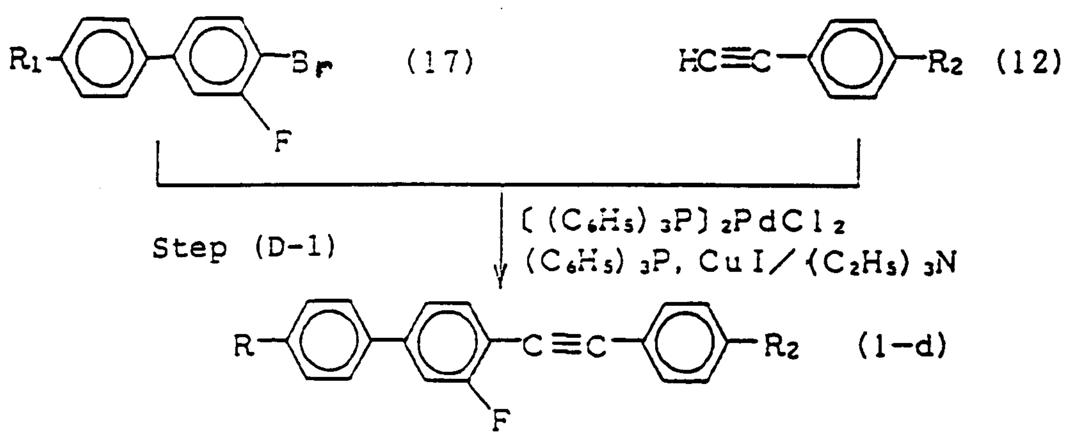


Step (D-1):

Compound (17) is reacted with compound (12) in triethylamine in the presence of bis(triphenylphosphine) palladium(II) chloride, triphenylphosphine and copper(I) iodide to obtain compound (1-d).

**Table 6**

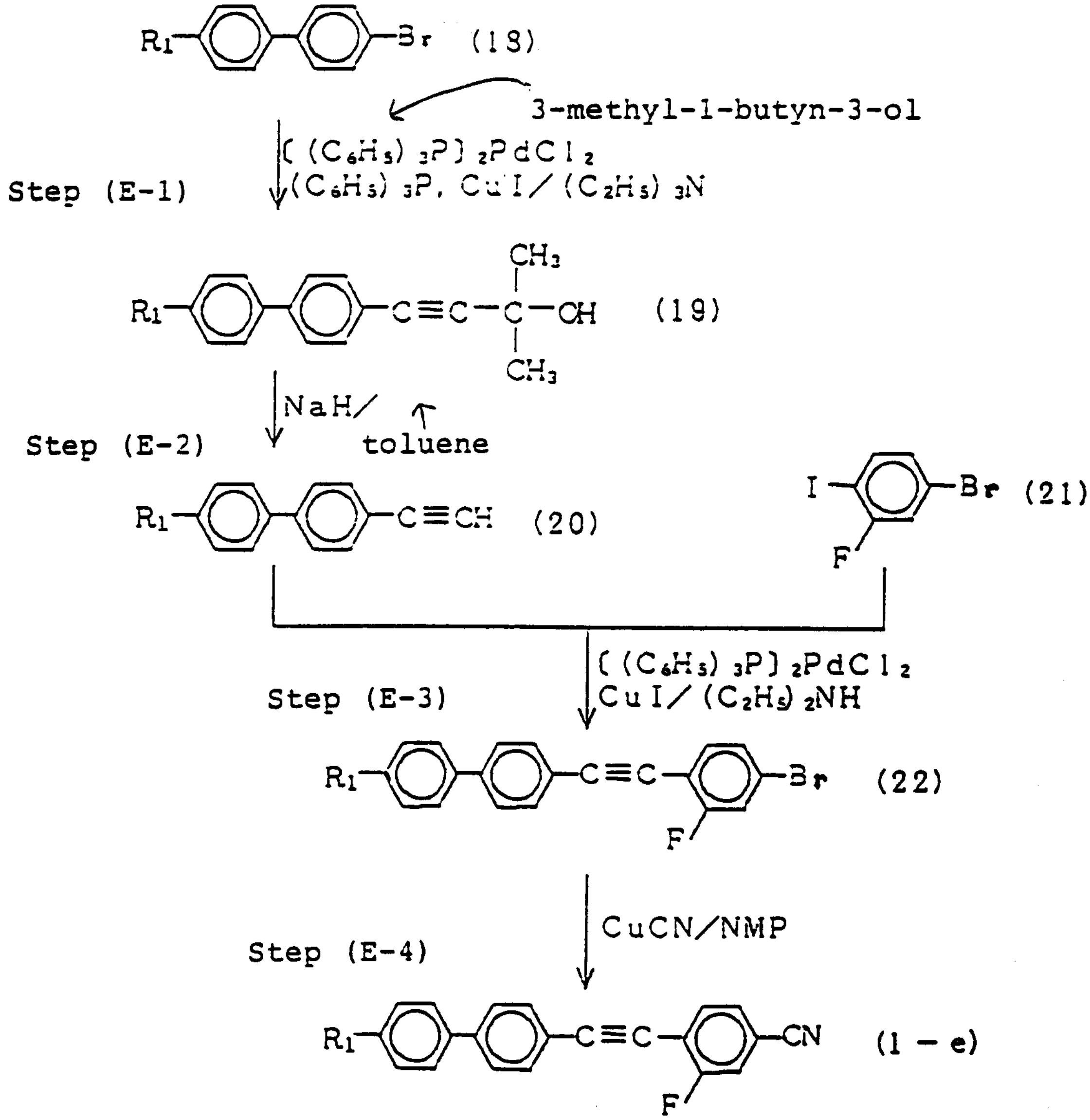


Step (E-1):

Compound (18) is reacted with 3-methyl-1-butyl-3-ol in triethylamine in the presence of bis(triphenylphosphine) palladium(II) chloride, triphenylphosphine and copper(I) iodide to obtain compound (19).

Step (E-2):

Compound (19) is reacted with sodium hydride in toluene to obtain compound (20).

Step (E-3):

Compound (20) is reacted with compound (21) in diethylamine in the presence of bis(triphenylphosphine) palladium(II) chloride and copper(I) iodide to obtain compound (22).

Step (E-4):

Compound (22) is reacted with copper(I) cyanide in N-methyl-pyrrolidone (hereinafter referred to as NMP) to obtain compound (1-e).

Table 7

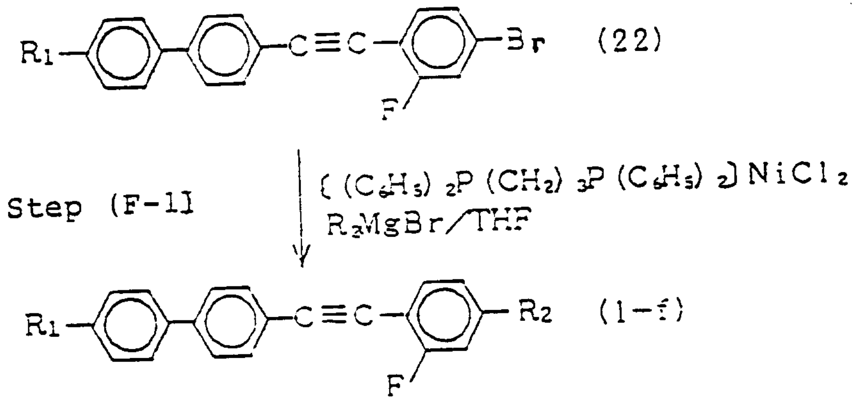


Step (F-1):

Compound (22) is reacted with a Grignard reagent prepared from an alkyl bromide, in THF in the presence of bis(1,3-diphenylphosphinopropane) nickel(II) chloride, to obtain compound (1-f).

Where the tolan derivatives of the present invention are blended to give a liquid crystal composition, the following compounds may be used as a base component of the composition. However, the illustrated compounds are not limitative. The tolan derivatives of the present invention are well compatible with all conventional liquid crystal compounds and their analogues, and the liquid crystal compositions to be obtained by blending them are characterized by having a broad practical temperature range and a large Δn value.

**Table 8**

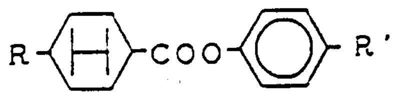

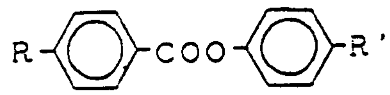

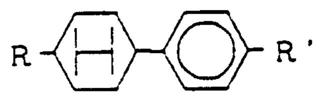

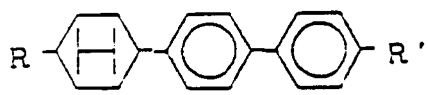

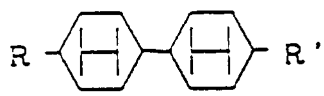

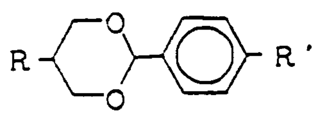

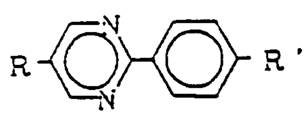

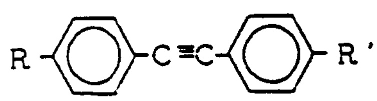

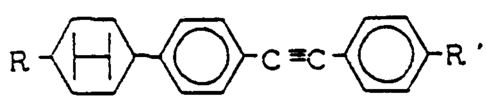

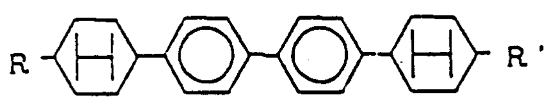

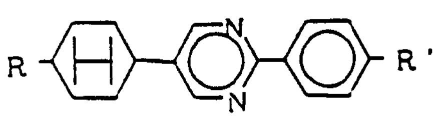

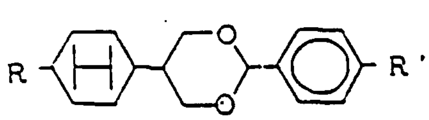

In these formulae, R' represents an alkyl group, an alkoxy group, an alkoxymethylene group, a nitrile group, or a fluoro group, the phenylene group may have a halogen substituent on the 2- or 3-position, and the cyclohexane ring is in the trans-configuration.

The proportion of the compound of the present invention contained in the liquid crystal composition may be from 1 to 50 % by weight and is especially preferably from 1 to 30 % by weight in consideration of precipitation of crystals in a low temperature range.

The most characteristic feature of the compounds of the present invention is the introduction of fluorine into the side position of known phenyltolan compounds (e.g., d-1, d-2) to noticeably lower the C-N point of them and to improve the compatibility of them with other liquid crystal compounds. Introduction of fluorine into the side position of liquid crystal compounds to lower the C-N point of them has heretofore been known, but the great depression of the C-N point achieved by the compounds of the present invention could not be anticipated. For instance, when compound (b-1) is compared with compound (c-1) and compound (b-2) with compound (c-2), the C-N point depression due to fluorine substitution is 17°C and 37°C, respectively. When compound (e-1) is compared with compound (e-2), compound (f-1) with compound (f-2) and compound (f-3) with compound (f-4), it is only 18°C, 17°C (28°C for Sm-N point depression) and 1°C (48°C for Sm-N point depression), respectively. On the contrary, in the case of the compounds of the present invention, where compound (g-1) is compared with compound (g-2), compound (g-1) with compared (g-3), compound (d-1) with compound (h-2) and compound (d-1) with compound (h-2), great C-N point depression of 49°C, 66°C, 79°C and 78°C, respectively, is noted. Due to the effect, practical use of phenyltolan derivatives, which have heretofore been difficult to put them into practical use, has become possible.

**Table 9**

| Code | Structural Formula | Transition Point | Reference |
|---|---|---|---|
| e-1 | 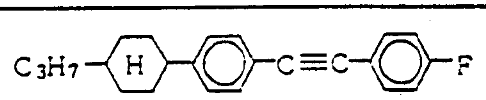 | C-N point 90°C<br>N-I point 189°C | JP-A 60-155142 |
| e-2 | 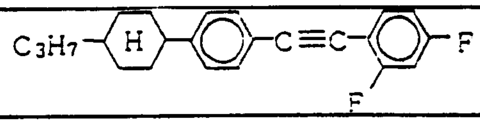 | C-N point 72°C<br>N-I point 163°C | JP-A 63-287737 |
| f-1 | 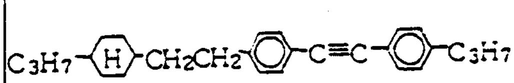 | C-Sm point 67°C<br>Sm-N point 78°C<br>N-I point 167°C | JP-A 63-152334 |
| f-2 | 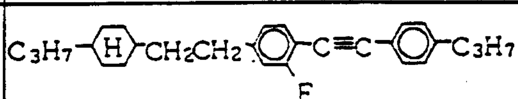 | C-N point 50°C<br>N-I point 156°C | JP-A 63-152334 |
| f-3 | 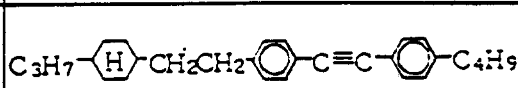 | C-Sm point 43°C<br>Sm-N point 90°C<br>N-I point 159°C | JP-A 63-152334 |
| f-4 | 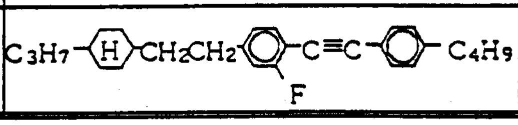 | C-N point 42°C<br>N-I point 149°C | JP-A 63-152334 |

Table 10

| Code | Structural Formura | Transition Point | Remarks |
|---|---|---|---|
| g-1 | 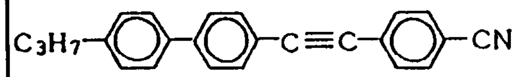 | C-Sm point 174°C<br>Sm-N point 180°C<br>N-I point 281°C | produced by the applicant |
| g-2 | 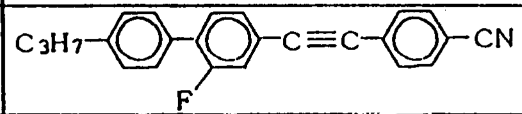 | C-N point 125°C<br>N-I point 238°C | compount (1-a) of the invention |
| g-3 | 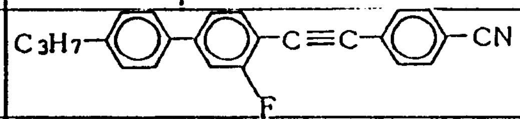 | C-N point 108°C<br>N-I point 256°C | compound (1-c) of the invention |
| g-4 | 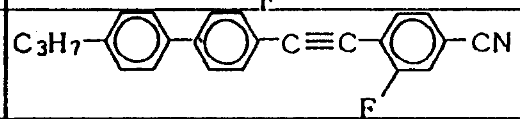 | C-Sm point 151°C<br>Sm-N point 185°C<br>N I point 268°C | compound (1-e) of the invention |
| d-1 | 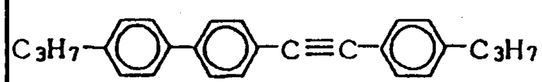 | C-N point 164°C<br>N-I point 230°C | JP-A 60-152427 |
| h-1 | 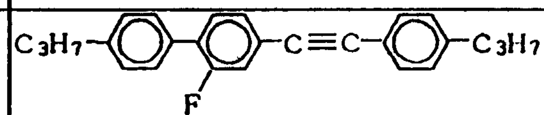 | C-N point 85°C<br>N-I point 176°C | compound (1-b) of the invention |
| h-2 | 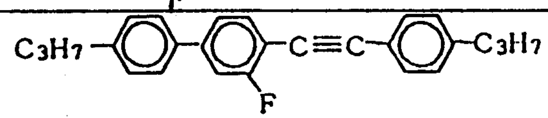 | C-N point 86°C<br>N-I point 197°C | compound (1-d) of the invention |
| h-3 | 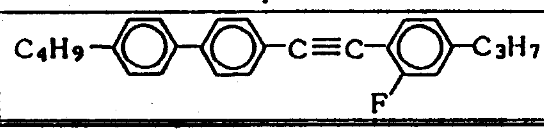 | C-Sm point 120°C<br>Sm-N point 163°C<br>S-I point 198°C | compound (1-f) of the invention |

A liquid crystal display device having a liquid crystal composition containing at least one compound of the present invention is suitable as a multiplex driving system device. Using it, in particular, high multiplex driving is possible in TN type and STN type liquid crystal display devices.

In the following the invention will be explained in more detail with reference to specific examples and with reference to the drawing showing in Fig. 1 a liquid crystal display cell.

Examples:

Next, the present invention will be explained in more detail by way of the following examples.

Example 1:

Production of Compound (1-a)

Production of 3-fluoro-4-(4"-propylphenyl)-4'-cyanotolan:

Step (A-1):

40 g of aluminum chloride and 280 ml of carbon disulfide were taken into a flask, cooled with an ice-salt bath to 0°C or lower and stirred. A solution of 70 g of 4-bromo-2-fluorobiphenyl in 180 ml of carbon disulfide was dropwise added thereto. A solution of 26 g of propionic acid chloride in 100 ml of carbon disulfide was dropwise added thereto at 0°C or lower. After addition, the whole was stirred for 2 hours at 0°C or lower and then at room temperature overnight. After reaction, the reaction solution was poured into 30 ml of concentrated hydrochloric acid and 300 g of ice and extracted with chloroform. The organic layer was washed three times each with water, two times each with an aqueous 2 % sodium hydroxide solution and then three times each with water, and chloroform and carbon disulfide were removed by distillation. The residue was recrystallized from a mixed solvent comprising acetone and methanol to obtain 29 g of 2-fluoro-4-bromo-4'-propionylbiphenyl.

Step (A-2):

29 g of 2-fluoro-4-bromo-4'-propionylbiphenyl was dissolved in 72 ml of trifluoroacetic acid and stirred. 24 g of triethylsilane was dropwise added thereto at room temperature over a period of 30 minutes. Then the mixture was stirred for 2 hours, 900 ml of an aqueous saturated sodium hydrogen-carbonate solution was added dropwise so that the excess trifluoroacetic acid was decomposed. This was extracted with chloroform and then washed three times each with water, and chloroform was removed by distillation. The residue was distilled under reduced pressure (b.p. 140 to 145°C/2 mmHg) to obtain 23.4 g of 2-fluoro-4-bromo-4'-propylbiphenyl.

Step (A-3):

30 g of 4-bromobenzonitrile, 13.4 g of 3-methyl-1-butyl-3-ol, 0.7 g of triphenylphosphine and 0.3 g of bis(triphenylphosphine) palladium(II) chloride were dissolved in 140 ml of triethylamine in nitrogen atmosphere, and 0.1 g of copper(I) iodide was added thereto. The mixture was stirred for one hour at room temperature and then for 5 hours at 90°C. The crystals as precipitated were filtered out and, after triethylamine was removed by distillation, extracted with chloroform. Then the crystals were washed two times each with 10 % hydrochloric acid and two times each with water, and chloroform was removed by distillation. The residue was distilled under reduced pressure (b.p. 140 to 160°C/5 mmHg) to obtain 23.5 g of 3-methyl-1-(4'-cyanophenyl)-1-butyl-3-ol.

Step (A-4):

23.5 g of 3-methyl-1-(4'-cyanophenyl)-1-butyl-3-ol was dissolved in 250 ml of toluene in nitrogen atmosphere, and 1.6 g of sodium hydride (60 % in paraffin liquid) was added thereto. This was stirred for 4 hours at 80 to 90°C. The reaction solution was poured into 300 ml of water, extracted with chloroform and washed three times each with water. Toluene and chloroform were removed by distillation, and the residue was recrystallized from methanol to obtain 9.9 g of 4-cyanophenylacetylene.

Step (A-5):

2.9 g of 2-fluoro-4-bromo-4'-propylbiphenyl, 1.3 g of 4-cyanophenylacetylene, 0.04 g of triphenylphosphine and 0.03 g of bis(triphenylphosphine) palladium(II) chloride were dissolved in 22 ml of triethylamine in nitrogen atmosphere, and 0.01 g of copper(I) iodide was added thereto. This was then refluxed for 5 hours. The reaction solution was poured into 200 ml of water, extracted with chloroform and washed two times each with water, and chloroform was removed by distillation. The residue was recrystallized from a mixed solvent comprising acetone and methanol, then purified by silicagel-chloroform column chromatography, and again recrystallized from acetone to obtain 1.9 g of 3-fluoro-4-(4"-propylphenyl)-4'-cyanotolan. The compound had a C-N point of 125.1°C and an N-I point of 237.6°C.

In the same manner, the following compounds were produced.

3-Fluoro-4-(4"-methylphenyl)-4'-cyanotolan
3-Fluoro-4-(4"-ethylphenyl)-4'-cyanotolan
C-N point 163.0°C, N-I point 235.9°C
3-Fluoro-4-(4"-butylphenyl)-4'-cyanotolan
C-N point 75.4°C, N-I point 220.7°C
3-Fluoro-4-(4"-pentylphenyl)-4'-cyanotolan
C-N point 85.6°C, N-I point 218.0°C
3-Fluoro-4-(4"-hexylphenyl)-4'-cyanotolan
3-Fluoro-4-(4"-heptylphenyl)-4'-cyanotolan
3-Fluoro-4-(4"-octylphenyl)-4'-cyanotolan
3-Fluoro-4-(4"-nonylphenyl)-4'-cyanotolan
3-Fluoro-4-(4"-decylphenyl)-4'-cyanotolan

Example 2:

Production of Compound (1-b):

Production of 3-fluoro-4-(4"-pentylphenyl)-4'-propyltolan:

Step (B-1):

294 g of aluminum chloride was added to 1000 ml of carbon disulfide and stirred. The reaction solution was cooled to 0°C or lower, and 166 g of propionyl chloride was added dropwise. Next, 283 g of bromobenzene was added dropwise. Then, the whole was stirred for one hour at room temperature. After reaction, the reaction solution was poured into a mixture of 300 ml of concentrated hydrochloric acid and 600 g of ice. This was extracted with chloroform and washed three times each with water, and chloroform was removed by distillation. By distillation under reduced pressure (b.p. 100 to 105°C/2 mmHg), 301 g of 1-bromo-4-propionylbenzene was obtained.

Step (B-2):

301 g of 1-bromo-4-propionylbenzene was dissolved in 1100 ml of diethylene glycol, and 145 ml of hydrazine (monohydrate) and 163 g of potassium hydroxide were added thereto and refluxed for one hour at 130°C. With removing water and hydrazine, this was heated up to 190°C. After having been refluxed for 5 hours at 190°C, this was poured into 2000 ml of water. This was extracted with chloroform and washed three times each with water, and chloroform was removed by distillation. By distillation under reduced pressure (b.p. 70 to 75°C/3 mmHg), 252 g of 1-bromo-4-propylbenzene was obtained.

Step (B-3):

60 g of 1-bromo-4-propylbenzene, 38 g of 3-methyl-1-butyl-3-ol, 1.3 g of tripheylphosphine and 0.7 g of bis(triphenylphosphine) palladium(II) chloride were dissolved in 260 ml of triethylamine in nitrogen atmosphere, and 0.2 g of copper(I) iodide was added thereto. This was stirred for one hour at room temperature and for 5 hours at 90°C. The crystals as precipitated were filtered out and, after triethylamine was removed by distillation, extracted with chloroform. The crystals were washed two times each with 10 % hydrochloric acid and two times each with water, and chloroform was removed by distillation. The residue was purified by silicagel-chloroform column chromatography to obtain 37 g of 3-methyl-1-(4'-propylphenyl)-1-butyl-3-ol.

Step (B-4):

33 g of 3-methyl-1-(4'-propylphenyl)-1-butyl-3-ol was dissolved in 320 ml of toluene in nitrogen atmosphere, and 2 g of sodium hydride (60 % in paraffin liquid) was added thereto. This was stirred for 6 hours at 60°C. The reaction solution was poured into 300 ml of water, extracted with chloroform and washed three times each with water. Toluene and chloroform were removed by distillation, and the residue was distilled under reduced pressure (b.p. 60 to 63°C/4 mmHg) to obtain 16 g of 4-propylphenylacetylene.

Step (B-5):

4 g of 4'-pentyl-2-fluoro-4-bromobiphenyl, 2.2 g of 4-propylphenylacetylene, 1.05 g of triphenylphosphine and 0.03 g of bis(triphenylphosphine) palladium(II) chloride were dissolved in 27 ml of triethylamine in nitrogen atmosphere, and 0.01 g of copper(I) iodide was added thereto. This was stirred for one hour at room temperature and then for 5 hours at 70°C. The reaction solution was poured into 300 ml of water, extracted with chloroform and washed two times each with water, and chloroform was removed by distillation. The residue was purified by silicagel-chloroform column chromatography and recrystallized from a mixed solvent comprising acetone and methanol to obtain 2.7 g of 3-fluoro-4-(4"-pentylphenyl)-4'-propyltolan. The compound had a C-N point of 74.7°C and an N-I point of 159.2°C.

In the same manner, the following compounds were produced.

3-Fluoro-4-(4"-methylphenyl)-4'-methyltolan
3-Fluoro-4-(4"-ethylphenyl)-4'-methyltolan
3-Fluoro-4-(4"-propylphenyl)-4'-methyltolan
3-Fluoro-4-(4"-butylphenyl)-4'-methyltolan
3-Fluoro-4-(4"-pentylphenyl)-4'-methyltolan
3-Fluoro-4-(4"-hexylphenyl)-4'-methyltolan
3-Fluoro-4-(4"-heptylphenyl)-4'-methyltolan
3-Fluoro-4-(4"-ocytlphenyl)-4'-methyltolan
3-Fluoro-4-(4"-nonylphenyl)-4'-methyltolan
3-Fluoro-4-(4"-decylphenyl)-4'-methyltolan
3-Fluoro-4-(4"-methylphenyl)-4'-ethyltolan
3-Fluoro-4-(4"-ethylphenyl)-4'-ethyltolan
C-N point 121.3°C, N-I point 154.0°C
3-Fluoro-4-(4"-propylphenyl)-4'-ethyltolan
C-N point 105.9°C, N-I point 166.9°C
3-Fluoro-4-94"-butylphenyl)-4'-ethyltolan
C-N point 76.6°C, N-I point 150.6°C
3-Fluoro-4-(4"-pentylphenyl)-4'-ethyltolan
C-N point 78.9°C, N-I point 154.8°C
3-Fluoro-4-(4"-hexylphenyl)-4'-ethyltolan
3-Fluoro-4-(4"-heptylphenyl)-4'-ethyltolan
3-Fluoro-4-(4"-octylphenyl)-4'-ethyltolan
3-Fluoro-4-(4"-nonylphenyl)-4'-ethyltolan
3-Fluoro-4-(4"-decylphenyl)-4'-ethyltolan
3-Fluoro-4-(4"-methylphenyl)-4'-propyltolan
3-Fluoro-4-(4"-ethylphenyl)-4'-propyltolan
C-N point 92.9°C, N-I point 165.2°C
3-Fluoro-4-(4"-propylphenyl)-4'-propyltolan
C-N point 84.2°C, N-I point 175.5°C
3-Fluoro-4-(4"-butylphenyl)-4'-propyltolan
C-N point 74.3°C, N-I point 160.0°C
3-Fluoro-4-(4"-hexylphenyl)-4'-propyltolan
3-Fluoro-4-(4"-heptylphenyl)-4'-propyltolan
3-Fluoro-4-(4"-octylphenyl)-4'-propyltolan
3-Fluoro-4-(4"-nonylphenyl)-4'-propyltolan
3-Fluoro-4-(4"-decylphenyl)-4'-propyltolan
3-Fluoro-4-(4"-methylphenyl)-4'-butyltolan
3-Fluoro-4-(4"-ethylphenyl)-4'-butyltolan
C-N point 61.2°C, N-I point 149.7°C
3-Fluoro-4-(4"-propylphenyl)-4'-butyltolan
C-N point 60.2°C, N-I point 160.1°C
3-Fluoro-4-(4"-butylphenyl)-4'-butyltolan
C-N point 68.1°C, N-I point 145.9°C
3-Fluoro-4-(4"-pentylphenyl)-4'-butyltolan
C-N point 56.6°C, N-I point 149.5°C
3-Fluoro-4-(4"-hexylphenyl)-4'-butyltolan
3-Fluoro-4-(4"-heptylphenyl)-4'-butyltolan
3-Fluoro-4-(4"-octylphenyl)-4'-butyltolan
3-Fluoro-4-(4"-nonylphenyl)-4'-butyltolan

3-Fluoro-4-(4''-decylphenyl)-4'-butyltolan
3-Fluoro-4-(4''-methylphenyl)-4'-pentyltolan
3-Fluoro-4-(4''-ethylphenyl)-4'-pentyltolan
C-N point 61.3°C, N-I point 152.4°C
3-Fluoro-4-(4''-propylphenyl)-4'-pentyltolan
C-N point 65.4°C, N-I point 162.8°C
3-Fluoro-4-(4''-butylphenyl)-4'-pentyltolan
C-N point 44.7°C, N-I point 150.7°C
3-Fluoro-4-(4''-pentylphenyl)-4'-pentyltolan
C-N point 44.4°C, N-I point 152.8°C
3-Fluoro-4-(4''-hexylphenyl)-4'-pentyltolan
3-Fluoro-4-(4''-heptylphenyl)-4'-pentyltolan
3-Fluoro-4-(4''-octylphenyl)-4'-pentyltolan
3-Fluoro-4-(4''-nonylphenyl)-4'-pentyltolan
3-Fluoro-4-(4''-decylphenyl)-4'-pentyltolan
3-Fluoro-4-(4''-methylphenyl)-4'-hexyltolan
3-Fluoro-4-(4''-ethylphenyl)-4'-hexyltolan
3-Fluoro-4-(4''-propylphenyl)-4'-hexyltolan
3-Fluoro-4-(4''-butylphenyl)-4'-hexyltolan
3-Fluoro-4-(4''-pentylphenyl)-4'-hexyltolan
3-Fluoro-4-(4''-hexylphenyl)-4'-hexyltolan
3-Fluoro-4-(4''-heptylphenyl)-4'-hexyltolan
3-Fluoro-4-(4''-octylphenyl)-4'-hexyltolan
3-Fluoro-4-(4''-nonylphenyl)-4'-hexyltolan
3-Fluoro-4-(4''-decylphenyl)-4'-hexyltolan
3-Fluoro-4-(4''-methylphenyl)-4'-heptyltolan
3-Fluoro-4-(4''-ethylphenyl)-4'-heptyltolan
3-Fluoro-4-(4''-propylphenyl)-4'-heptyltolan
3-Fluoro-4-(4''-butylphenyl)-4'-heptyltolan
3-Fluoro-4-(4''-pentylphenyl)-4'-heptyltolan
3-Fluoro-4-(4''-hexylphenyl)-4'-heptyltolan
3-Fluoro-4-(4''-heptylphenyl)-4'-heptyltolan
3-Fluoro-4-(4''-octylphenyl)-4'-heptyltolan
3-Fluoro-4-(4''-nonylphenyl)-4'-heptyltolan
3-Fluoro-4-(4''-decylphenyl)-4'-heptyltolan
3-Fluoro-4-(4''-methylphenyl)-4'-octyltolan
3-Fluoro-4-(4''-ethylphenyl)-4'-octyltolan
3-Fluoro-4-(4''-propylphenyl)-4'-octyltolan
3-Fluoro-4-(4''-butylphenyl)-4'-octyltolan
3-Fluoro-4-(4''-pentylphenyl)-4'-octyltolan
3-Fluoro-4-(4''-hexylphenyl)-4'-octyltolan
3-Fluoro-4-(4''-heptylphenyl)-4'-octyltolan
3-Fluoro-4-(4''-octylphenyl)-4'-octyltolan
3-Fluoro-4-(4''-nonylphenyl)-4'-octyltolan
3-Fluoro-4-(4''-decylphenyl)-4'-octyltolan
3-Fluoro-4-(4''-methylphenyl)-4'-nonyltolan
3-Fluoro-4-(4''-ethylphenyl)-4'-nonyltolan
3-Fluoro-4-(4''-propylphenyl)-4'-nonyltolan
3-Fluoro-4-(4''-butylphenyl)-4'-nonyltolan
3-Fluoro-4-(4''-pentylphenyl)-4'-nonyltolan
3-Fluoro-4-(4''-hexylphenyl)-4'-nonyltolan
3-Fluoro-4-(4''-heptylphenyl)-4'-nonyltolan
3-Fluoro-4-(4''-octylphenyl)-4'-nonyltolan
3-Fluoro-4-(4''-nonylphenyl)-4'-nonyltolan
3-Fluoro-4-(4''-decylphenyl)-4'-nonyltolan
3-Fluoro-4-(4''-methylphenyl)-4'-decyltolan
3-Fluoro-4-(4''-ethylphenyl)-4'-decyltolan
3-Fluoro-4-(4''-propylphenyl)-4'-decyltolan
3-Fluoro-4-(4''-butylphenyl)-4'-decyltolan

3-Fluoro-4-(4"-pentylphenyl)-4'-decyltolan
3-Fluoro-4-(4"-hexylphenyl)-4'-decyltolan
3-Fluoro-4-(4"-heptylphenyl)-4'-decyltolan
3-Fluoro-4-(4"-octylphenyl)-4'-decyltolan
3-Fluoro-4-(4"-nonylphenyl)-4'-decyltolan
3-Fluoro-4-(4"-decylphenyl)-4'-decyltolan

Example 3:

Production of Compound (1-c):

Production of 2-fluoro-4-(4"-propylphenyl)-4'-cyanotolan:

Step (C-1):

9.3 g of magnesium was put into a flask in nitrogen atmosphere, and a solution of 68 g of 4-propyl-1-bromobenzene in 350 ml of THF was added dropwise. After addition, this was stirred at room temperature overnight to form a Grignard reagent. 70 g of trimethyl borate was dissolved in 38 ml of THF, and the Grignard reagent was dropwise added thereto at room temperature. After addition, this was stirred at room temperature overnight. Next, 75 ml of 10 % hydrochloric acid was added thereto and stirred for one hour. This was extracted with chloroform and washed three times each with water, and chloroform was removed by distillation to obtain 41 g of 4-propylphenylboric acid.

Step (C-2):

51.2 g of sodium nitrite was dissolved in 390 ml of sulfuric acid, and 454 ml of acetic acid was added thereto at 10°C or lower. The solution was kept at 20 to 25°C, and 124 g of 2-fluoro-4-iodoaniline was added thereto over a period of one hour and stirred for 2 hours. The solution was added dropwise to a solution of 130 g of copper(I) bromide in 390 ml of 48 % hydrobromic acid and stirred overnight. Next, 1000 ml of water was added to this, which was then extracted with chloroform and washed three times each with water. Chloroform was removed by distillation, and the residue was distilled under reduced pressure (b.p. 120 to 125°C/13 mmHg) and recrystallized from methanol to obtain 114 g of 1-bromo-2-fluoro-4-iodobenzene.

Step (C-3):

A solution of 16.5 g of 4-propylphenylboric acid in 130 ml of ethanol was added dropwise to a mixture comprising 30 g of 1-bromo-2-fluoro-4-iodobenzene, 1.6 g of tetrakistriphenylphosphine palladium(0), 186 ml of benzene and 140 ml of 2M aqueous sodium carbonate solution, in nitrogen atmosphere at room temperature. The reaction solution was then refluxed for 5 hours, cooled to room temperature, extracted with chloroform and washed three times each with water, and chloroform was removed by distillation. The residue formed was distilled under reduced pressure (b.p. 155 to 170°C/3 mmHg) to obtain 11.7 g of 4-bromo-3-fluoro-4'-propylbiphenyl.

Step (C-4):

2.6 g of 4-bromo-3-fluoro-4'-propylbiphenyl, 1.1 g of 4-cyanophenylacetylene, 0.03 g of triphenylphosphine and 0.03 g of bis(triphenylphosphine) palladium(II) chloride were dissolved in 20 ml of triethylamine in nitrogen atmosphere, and 0.01 g of copper(I) iodide was added thereto. This was then refluxed for 5 hours. The reaction solution was poured into 200 ml of water, extracted with chloroform and washed two times each with water, and chloroform was removed by distillation. The residue was recrystallized from a mixed solvent comprising acetone and methanol, purified by silicagel-chloroform-column chromatography and again recrystallized from a mixed solvent of acetone and methanol to obtain 0.8 g of 2-fluoro-4-(4"-propylphenyl)-4'-cyanotolan. The compound had a C-N point of 108.0°C and an N-I point of 256.1°C.

In the same manner, the following compounds were produced.

2-Fluoro-4-(4"-methylphenyl)-4'-cyanotolan
2-Fluoro-4-(4"-ethylphenyl)-4'-cyanotolan
2-Fluoro-4-(4"-butylphenyl)-4'-cyanotolan
C-N point 95.0°C, N-I point 240.0°C
2-Fluoro-4-(4"-pentylphenyl)-4'-cyanotolan
C-N point 89.0°C, N-I point 233.5°C
2-Fluoro-4-(4"-hetylphenyl)-4'-cyanotolan

2-Fluoro-4-(4"-heptylphenyl)-4'-cyanotolan
2-Fluoro-4-(4"-octylphenyl)-4'-cyanotolan
2-Fluoro-4-(4"-nonylphenyl)-4'-cyanotolan
2-Fluoro-4-(4"-deyclphenyl)-4'-cyanotolan

Example 4:

Production of Compound (1-d):

Step (D-1):

4.6 g of 4-bromo-3-fluoro-4'-butylbiphenyl, 2.6 g of 4-pentylphenylacetylene, 0.06 g of triphenylphosphine and 0.04 g of bis(triphenylphosphine) palladium(II) chloride were dissolved in 35 ml of triethylamine in nitrogen atmosphere, and 0.01 g of copper(I) iodide was added thereto. This was stirred for one hour at room temperature and then for 5 hours at 70°C. The reaction solution was poured into 300 ml of water, extracted with chloroform and washed two times each with water, and chloroform was removed by distillation. The residue was purified by silicagel-chloroform column chromatography and recrystallized from a mixed solvent comprising acetone and methanol to obtain 1.3 g of 2-fluoro-4-(4"-butylphenyl)-4'-pentyltolan. The compound had a C-N point of 50.5°C and an N-I point of 171.2°C.

In the same manner, the following compounds were produced.

2-Fluoro-4-(4"-methylphenyl)-4'-methyltolan
2-Fluoro-4-(4"-ethylphenyl)-4'-methyltolan
2-Fluoro-4-(4"-propylphenyl)-4'-methyltolan
2-Fluoro-4-(4"-butylphenyl)-4'-methyltolan
2-Fluoro-4-(4"-pentylphenyl)-4'-methyltolan
2-Fluoro-4-(4"-hexylphenyl)-4'-methyltolan
2-Fluoro-4-(4"-heptylphenyl)-4'-methyltolan
2-Fluoro-4-(4"-octylphenyl)-4'-methyltolan
2-Fluoro-4-(4"-nonylphenyl)-4'-methyltolan
2-Fluoro-4-(4"-decylphenyl)-4'-methyltolan
2-Fluoro-4-(4"-methylphenyl)-4'-ethyltolan
2-Fluoro-4-(4"-ethylphenyl)-4'-ethyltolan
2-Fluoro-4-(4"-propylphenyl)-4'-ethyltolan
2-Fluoro-4-(4"-butylphenyl)-4'-ethyltolan
2-Fluoro-4-(4"-pentylphenyl)-4'-ethyltolan
2-Fluoro-4-(4"-hexylphenyl)-4'-ethyltolan
2-Fluoro-4-(4"-heptylphenyl)-4'-ethyltolan
2-Fluoro-4-(4"-octylphenyl)-4'-ethyltolan
2-Fluoro-4-(4"-nonylphenyl)-4'-ethyltolan
2-Fluoro-4-(4"-decylphenyl)-4'-ethyltolan
2-Fluoro-4-(4"-methylphenyl)-4'-propyltolan
2-Fluoro-4-(4"-ethylphenyl)-4'-propyltolan
2-Fluoro-4-(4"-propylphenyl)-4'-propyltolan
C-N point 85.7°C, N-I point 196.7°C
2-Fluoro-4-(4"-butylphenyl)-4'-propyltolan
C-N point 69.9°C, N-I point 182.7°C
2-Fluoro-4-(4"-pentylphenyl)-4'-propyltolan
C-N point 79.7°C, N-I point 184.7°C
2-Fluoro-4-(4"-hexylphenyl)-4'-propyltolan
2-Fluoro-4-(4"-heptylphenyl)-4'-propyltolan
2-Fluoro-4-(4"-octylphenyl)-4'-propyltolan
2-Fluoro-4-(4"-nonylphenyl)-4'-propyltolan
2-Fluoro-4-(4"-decylphenyl)-4'-propyltolan
2-Fluoro-4-(4"-methylphenyl)-4'-butyltolan
2-Fluoro-4-(4"-ethylphenyl)-4'-butyltolan
2-Fluoro-4-(4"-propylphenyl)-4'-butyltolan
2-Fluoro-4-(4"-butylphenyl)-4'-butyltolan
C-N point 63.7°C, N-I point 169.4°C
2-Fluoro-4-(4"-pentylphenyl)-4'-butyltolan
2-Fluoro-4-(4"-hexylphenyl)-4'-butyltolan

2-Fluoro-4-(4"-heptylphenyl)-4'-butyltolan
2-Fluoro-4-(4"-octylphenyl)-4'-butyltolan
2-Fluoro-4-(4"-nonylphenyl)-4'-butyltolan
2-Fluoro-4-(4"-decylphenyl-4'-butyltolan
2-Fluoro-4-(4"-methylphenyl)-4'-pentyltolan
2-Fluoro-4-(4"-ethylphenyl)-4'-pentyltolan
2-Fluoro-4-(4"-propylphenyl)-4'-pentyltolan
C-N point 78.3°C, N-I point 184.9°C
2-Fluoro-4-(4"-pentylphenyl)-4'-pentyltolan
2-Fluoro-4-(4"-hexylphenyl)-4'-pentyltolan
2-Fluoro-4-(4"-heptylphenyl)-4'-pentyltolan
2-Fluoro-4-(4"-octylphenyl)-4'-pentyltolan
2-Fluoro-4-(4"-nonylphenyl)-4'-pentyltolan
2-Fluoro-4-(4"-decylphenyl)-4'-pentyltolan
2-Fluoro-4-(4"-methylphenyl)-4'-hexyltolan
2-Fluoro-4-(4"-ethylphenyl)-4'-hexyltolan
2-Fluoro-4-(4"-propylphenyl)-4'-hexyltolan
2-Fluoro-4-(4"-butylphenyl)-4'-hexyltolan
2-Fluoro-4-(4"-pentylphenyl)-4'-hexyltolan
2-Fluoro-4-(4"-hexylphenyl)-4'-hexyltolan
2-Fluoro-4-(4"-heptylphenyl)-4'-hexyltolan
2-Fluoro-4-(4"-octylphenyl)-4'-hexyltolan
2-Fluoro-4-(4"-nonylphenyl)-4'-hexyltolan
2-Fluoro-4-(4"-decylphenyl)-4'-hexyltolan
2-Fluoro-4-(4"-methylphenyl)-4'-heptyltolan
2-Fluoro-4-(4"-ethylphenyl)-4'-heptyltolan
2-Fluoro-4-(4"-propylphenyl)-4'-heptyltolan
2-Fluoro-4-(4"-butylphenyl-4'-heptyltolan
2-Fluoro-4-(4"-pentylphenyl)-4'-heptyltolan
2-Fluoro-4-(4"-hexylphenyl)-4'-heptyltolan
2-Fluoro-4-(4"-heptylphenyl)-4'-heptyltolan
2-Fluoro-4-(4"-octylphenyl)-4'-heptyltolan
2-Fluoro-4-(4"-nonylphenyl)-4'-heptyltolan
2-Fluoro-4-(4"-decylphenyl)-4'-heptyltolan
2-Fluoro-4-(4"-methylphenyl)-4'-octyltolan
2-Fluoro-4-(4"-ethylphenyl)-4'-octyltolan
2-Fluoro-4-(4"-propylphenyl)-4'-octyltolan
2-Fluoro-4-(4"-butylphenyl)-4'-octyltolan
2-Fluoro-4-(4"-pentylphenyl)-4'-octyltolan
2-Fluoro-4-(4"-hexylphenyl)-4'-octyltolan
2-Fluoro-4-(4"-heptylphenyl)-4'-octyltolan
2-Fluoro-4-(4"-octylphenyl)-4'-octyltolan
2-Fluoro-4-(4"-nonylphenyl)-4'-octyltolan
2-Fluoro-4-(4"-decylphenyl)-4'-octyltolan
2-Fluoro-4-(4"-methylphenyl)-4'-nonyltolan
2-Fluoro-4-(4"-ethylphenyl)-4'-nonyltolan
2-Fluoro-4-(4"-propylphenyl)-4'-nonyltolan
2-Fluoro-4-(4"-butylphenyl)-4'-nonyltolan
2-Fluoro-4-(4"-pentylphenyl)-4'-nonyltolan
2-Fluoro-4-(4"-hexylphenyl)-4'-nonyltolan
2-Fluoro-4-(4"-heptylphenyl)-4'-nonyltolan
2-Fluoro-4-(4"-octylphenyl)-4'-nonyltolan
2-Fluoro-4-(4"-nonylphenyl)-4'-nonyltolan
2-Fluoro-4-(4"-decylphenyl)-4'-nonyltolan
2-Fluoro-4-(4"-methylphenyl)-4'-decyltolan
2-Fluoro-4-(4"-ethylphenyl)-4'-decyltolan
2-Fluoro-4-(4"-propylphenyl)-4'-decyltolan
2-Fluoro-4-(4"-butylphenyl)-4'-decyltolan
2-Fluoro-4-(4"-pentylphenyl)-4'-decyltolan

2-Fluoro-4-(4"-hexylphenyl)-4'-decyltolan
2-Fluoro-4-(4"-heptylphenyl)-4'-decyltolan
2-Fluoro-4-(4"-octylphenyl)-4'-decyltolan
2-Fluoro-4-(4"-nonylphenyl)-4'-decyltolan
2-Fluoro-4-(4"-decylphenyl)-4'-decyltolan

Example 5:

Production of Compound (1-e):

Production of 4-(4"-pentylphenyl)-2'-fluoro-4'-cyanotolan:

Step (E-1):

87 g of 1-bromo-4'-pentylbiphenyl, 36 g of 3-methyl-1-butyl-3-ol, 1.2 g of triphenylphosphine and 0.62 g of bis(triphenylphosphine) palladium(II) chloride were dissolved in 300 ml of triethylamine in nitrogen atmosphere, and 0.2 g of copper(I) iodide was added thereto. This was stirred at room temperature for one hour and then at 90°C for 5 hours. The reaction solution was poured into water, and the crystals as precipitated were filtered out and washed with water. The crystals thus obtained were dissolved in chloroform and washed two times each with 10 % hydrochloric acid and two times each with water, and chloroform was removed by distillation.

The residue was recrystallized from a mixed solvent of acetone and methanol to obtain 37 g of 3-methyl-1-(4"-pentylbiphenyl-4-yl)-1-butyl-3-ol.

Step (E-2):

37 g of 3-methyl-1-(4"-pentylbiphenyl-4-yl)-1-butyl-3-ol was dissolved in 250 ml of toluene in nitrogen atmosphere, and 1.5 g of sodium hydride (60 % in paraffin liquid) was added thereto. This was stirred for 6 hours at 60°C. The reaction solution was poured into 1000 ml of water, extracted with chloroform and washed three times each with water. Toluene and chloroform were removed by distillation, and the residue was recrystallized from methanol to obtain 30 g of 4-ethynyl-4'-pentylbiphenyl.

Step (E-3):

3.6 g of 4-bromo-2-fluoro-1-iodobenzene was dissolved in 4.4 ml of diethylamine in nitrogen atmosphere, and 0.04 g of bis(triphenylphosphine) palladium(II) chloride and 0.04 g of copper(I) iodide were added thereto and stirred. The flask containing the reaction mixture was cooled to 5°C or lower, and a solution of 3 g of 4-ethynyl-4'-pentylbiphenyl as dissolved in 8 ml of diethylamine was dropwise added thereto. This was stirred at room temperature for 5 hours, and the reaction solution was poured into a mixture of 7 ml of concentrated hydrochloric acid and 100 g of ice. This was extracted with chloroform and washed two times each with water, and chloroform was removed by distillation. The residue was recrystallized from a mixed solvent comprising acetone and chloroform to obtain 3.2 g of 4-(4"-pentylphenyl)-2'-fluoro-4'-bromotolan.

Step (E-4):

3.2 g of 4-(4"-pentylphenyl)-2'-fluoro-4'-bromotolan and 1 g of copper(I) cyanide were added to 16 ml of NMP and refluxed for 2 hours. The reaction solution was poured into a mixture of 4 g of iron(II) chloride, 1.2 ml of concentrated hydrochloric acid and 4.6 ml of water. The crystals precipitated was filtered out and washed with water. The thus obtained crystals were dissolved in chloroform and washed three times each with water, and chloroform was removed by distillation. The residue was purified by silicagel-chloroform-column chromatography and recrystallized from a mixed solvent comprising acetone and methanol to obtain 1.5 g of 4-(4"-pentylphenyl)-2'-fluoro-4'-cyanotolan. The compound had a crystal smectic phase transition point (hereinafter referred to as C-Sm point) of 123.9°C, a smectic nematic phase transition point (hereinafter referred to as Sm-N point) of 167.3°C and an N-I point of 245.3°C.

In the same manner, the following compounds were produced.

4-(4"-Methylphenyl)-2'-fluoro-4'-cyanotolan
4-(4"-Ethylphenyl)-2'-fluoro-4'-cyanotolan
C-Sm point 137.4°C, Sm-N point 175.1°C, N-I point 269.0°C
4-(4"-Propylphenyl)-2'-fluoro-4'-cyanotolan
C-Sm point 150.6°C, Sm-N point 185.1°C, N-I point 268.4°C
4-(4"-Butylphenyl)-2'-fluoro-4'-cyanotolan

C-Sm point 120.9°C, Sm-N point 176.0°C, N-I point 250.1°C
4-(4"-Hexylphenyl)-2'-fluoro-4'-cyanotolan
4-(4"-heptylphenyl)-2'-fluoro-4'-cyanotolan
4-(4"-octylphenyl)-2'-fluoro-4'-cyanotolan
4-(4"-nonylphenyl)-2'-fluoro-4'-cyanotolan
4-(4"-decylphenyl)-2'-fluoro-4'-cyanotolan

Example 6:

Production of Compound (1-f):

Production of 4-(4"-pentylphenyl)-2'-fluoro-4'-propyltolan:

Step (F-1):

1.5 g of 1-bromopropane was dissolved in 20 ml of THF in nitrogen atmosphere and added dropwise to 0.3 g of magnesium metal. After addition, this was stirred for 3 hours at room temperature to prepare a Grignard reagent. In another flask, 0.12 g of bis(1,3-diphenylphosphinopropane) nickel (II) chloride and 4.2 g of 4-(4"-pentylphenyl)-2'-fluoro-4'-bromotolan were dissolved in 16 ml of THF, and the previously prepared Grignard reagent was added dropwise. This was stirred at room temperature overnight, and the reaction solution was poured into a mixture of 4 ml of concentrated hydrochloric acid and 32 g of ice. This was extracted with chloroform and washed three times each with water, and chloroform was removed by distillation. The residue was recrystallized from a mixed solvent comprising acetone and methanol, and the crystals thus obtained were purified by silicagel-chloroform-column chromatography and again recrystallized from a mixed solvent of acetone and methanol to obtain 1.6 g of 4-(4"-pentylpheny1)-2'-fluoro-4'-propyltolan. The compound has a C-Sm point of 131.0°C, an Sm-N point of 172.5°C and an N-I point of 198.3°C.

In the same manner, the following compounds were produced.

4-(4"-Methylphenyl)-2'-fluoro-4'-methyltolan
4-(4"-Ethylphenyl)-2'-fluoro-4'-methyltolan
4-(4"-Propylphenyl)-2'-fluoro-4'-methyltolan
4-(4"-Butylphenyl)-2'-fluoro-4'-methyltolan
4-(4"-Pentylphenyl)-2'-fluoro-4'-methyltolan
4-(4"-Hexylphenyl)-2'-fluoro-4'-methyltolan
4-(4"-Octylphenyl)-2'-fluoro-4'-methyltolan
4-(4"-Nonylphenyl)-2'-fluoro-4'-methyltolan
4-(4"-Decylphenyl)-2'-fluoro-4'-methyltolan
4-(4"-Decylphenyl)-2'-fluoro-4'-methyltolan
4-(4"-Methylphenyl)-2'-fluoro-4'-ethyltolan
4-(4"-Ethylphenyl)-2'-fluoro-4'-ethyltolan
4-(4"-Propylphenyl)-2'-fluoro-4'-ethyltolan
4-(4"-Butylphenyl)-2'-fluoro-4'-ethyltolan
4-(4"-Pentylphenyl)-2'-fluoro-4'-ethyltolan
4-(4"-Hexylphenyl)-2'-fluoro-4'-ethyltolan
4-(4"-Octylphenyl)-2'-fluoro-4'-ethyltolan
4-(4"-Nonylphenyl)-2'-fluoro-4'-ethyltolan
4-(4"-Decylphenyl)-2'-fluoro-4'-ethyltolan
4-(4"-Decylphenyl)-2'-fluoro-4'-ethyltolan
4-(4"-Methylphenyl)-2'-fluoro-4'-propyltolan
4-(4"-Ethylphenyl)-2'-fluoro-4'-propyltolan
C-N point 163.6°C, N-I point 196.7°C
4-(4"-Propylphenyl)-2'-fluoro-4'-propyltolan
C-Sm point 131.0°C, Sm-N point 172.5°C, N-I point 198.3°C
4-(4"-Hexylphenyl)-2'-fluoro-4'-propyltolan
4-(4"-Heptylphenyl)-2'-fluoro-4'-propyltolan
4-(4"-Octylphenyl)-2'-fluoro-4'-propyltolan
4-(4"-Nonylphenyl)-2'-fluoro-4'-propyltolan
4-(4"-Decylphenyl)-2'-fluoro-4'-propyltolan
4-(4"-Methylphenyl)-2'-fluoro-4'-butyltolan
4-(4"-Ethylphenyl)-2'-fluoro-4'-butyltolan
4-(4"-Propylphenyl)-2'-fluoro-4'-butyltolan

4-(4''-Butylphenyl)-2'-fluoro-4'-butyltolan
4-(4''-Pentylphenyl)-2'-fluoro-4'-butyltolan
4-(4''-Hexylphenyl)-2'-fluoro-4'-butyltolan
4-(4''-Heptylphenyl)-2'-fluoro-4'-butyltolan
4-(4''-Octylphenyl)-2'-fluoro-4'-butyltolan
4-(4''-Nonylphenyl)-2'-fluoro-4'-butyltolan
4-(4''-Decylphenyl)-2'-fluoro-4'-butyltolan
4-(4''-Methylphenyl)-2'-fluoro-4'-pentyltolan
4-(4''-Ethylphenyl)-2'-fluoro-4'-pentyltolan
4-(4''-Propylphenyl)-2'-fluoro-4'-pentyltolan
4-(4''-Butylphenyl)-2'-fluoro-4'-pentyltolan
4-(4''-Pentylphenyl)-2'-fluoro-4'-pentyltolan
4-(4''-Hexylphenyl)-2'-fluoro-4'-pentyltolan
4-(4''-Heptylphenyl)-2'-fluoro-4'-pentyltolan
4-(4''-Ocytlphenyl)-2'-fluoro-4'-pentyltolan
4-(4''-Nonylphenyl)-2'-fluoro-4'-pentyltolan
4-(4''-Decylphenyl)-2'-fluoro-4'-pentyltolan
4-(4''-Methylphenyl)-2'-fluoro-4'-hexyltolan
4-(4''-Ethylphenyl)-2'-fluoro-4'-hexyltolan
4-(4''-Propylphenyl)-2'-fluoro-4'-hexyltolan
4-(4''-Butylphenyl)-2'-fluoro-4'-hexyltolan
4-(4''-Pentylphenyl)-2'-fluoro-4'-hexyltolan
4-(4''-Hexylphenyl)-2'-fluoro-4'-hexyltolan
4-(4''-Heptylphenyl)-2'-fluoro-4'-hexyltolan
4-(4''-Octylphenyl)-2'-fluoro-4'-hexyltolan
4-(4''-Nonylphenyl)-2'-fluoro-4'-hexyltolan
4-(4''-Decylphenyl)-2'-fluoro-4'-hexyltolan
4-(4''-Methylphenyl)-2'-fluoro-4'-heptyltolan
4-(4''-Ethylphenyl)-2'-fluoro-4'-heptyltolan
4-(4''-Propylphenyl)-2'-fluoro-4'-heptyltolan
4-(4''-Butylphenyl)-2'-fluoro-4'-heptyltolan
4-(4''-pentylphenyl)-2'-fluoro-4'-heptyltolan
4-(4''-Hexylphenyl)-2'-fluoro-4'-heptyltolan
4-(4''-Heptylphenyl)-2'-fluoro-4'-heptyltolan
4-(4''-octylphenyl)-2'-fluoro-4'-heptyltolan
4-(4''-Nonylphenyl)-2'-fluoro-4'-heptyltolan
4-(4''-Decylphenyl)-2'-fluoro-4'-heptyltolan
4-(4''-Methylphenyl)-2'-fluoro-4'-octyltolan
4-(4''-Ethylphenyl)-2'-fluoro-4'-octyltolan
4-(4''-Propylphenyl)-2'-fluoro-4'-octyltolan
4-(4''-Butylphenyl)-2'-fluoro-4'-octyltolan
4-(4''-Pentylphenyl)-2'-fluoro-4'-octyltolan
4-(4''-Hexylphenyl)-2'-fluoro-4'-octyltolan
4-(4''-Heptylphenyl)-2'-fluoro-4'-octyltolan
4-(4''-Ocytlphenyl)-2'-fluoro-4'-octyltolan
4-(4''-Nonylphenyl)-2'-fluoro-4'-octyltolan
4-(4''-Decylphenyl)-2'-fluoro-4'-octyltolan
4-(4''-Methylphenyl)-2'-fluoro-4'-nonyltolan
4-(4''-Ethylphenyl)-2'-fluoro-4'-nonyltolan
4-(4''-Propylphenyl)-2'-fluoro-4'-nonyltolan
4-(4''-Butylphenyl)-2'-fluoro-4'-nonyltolan
4-(4''-Pentylphenyl)-2'-fluoro-4'-nonyltolan
4-(4''-Hexylphenyl)-2'-fluoro-4'-nonyltolan
4-(4''-Heptylphenyl)-2'-fluoro-4'-nonyltolan
4-(4''-Octylphenyl)-2'-fluoro-4'-nonyltolan
4-(4''-Nonylphenyl)-2'-fluoro-4'-nonyltolan
4-(4''-Decylphenyl)-2'-fluoro-4'-nonyltolan
4-(4''-Methylphenyl)-2-fluoro-4-decyltolan
4-(4''-Ethylphenyl)-2'-fluoro-4'-decyltolan

4-(4"-Propylphenyl)-2'-fluoro-4'-decyltolan
4-(4"-Butylphenyl)-2'-fluoro-4'-decyltolan
4-(4"-Pentylphenyl)-2'-fluoro-4'-decyltolan
4-(4"-Hexylphenyl)-2'-fluoro-4'-decyltolan
4-(4"-Heptylphenyl)-2'-fluoro-4'-decyltolan
4-(4"-Octylphenyl)-2'-fluoro-4'-decyltolan
4-(4"-Nonylphenyl)-2'-fluoro-4'-decyltolan
4-(4"-Decylphenyl)-2'-fluoro-4'-decyltolan

Example 7:

Liquid Crystal Composition

Liquid crystal compositions (a) to (g) of the present invention were prepared, each containing ECH liquid crystals as base liquid crystals and compounds of the present invention as obtained in Examples 1 to 6 in the proportion mentioned below. For comparison, comparative liquid crystal compositions (h) to (j) were also prepared, each containing only known liquid crystal compounds in the proportion mentioned below.

**Table 11**

| Liquid Crystal Composition | a | b | c |
|---|---|---|---|
| 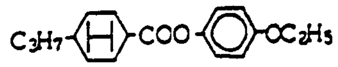 | 4.6 | 5.7 | 5.7 |
| 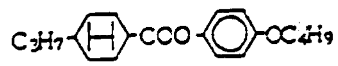 | 11.8 | 14.7 | 14.7 |
| 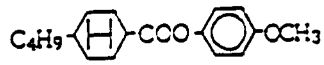 | 9.2 | 11.5 | 11.5 |
| 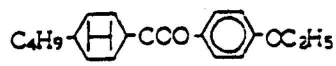 | 9.3 | 11.5 | 11.5 |
| 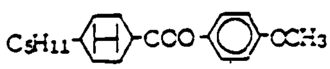 | 10.1 | 12.6 | 12.6 |
| 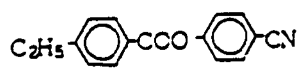 | 14.0 | 12.0 | 12.0 |
| 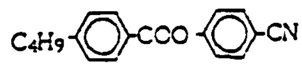 | 14.0 | | 6.0 |
| 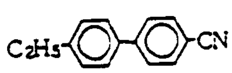 | 6.0 | 12.0 | 6.0 |
| 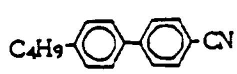 | 6.0 | | |
| 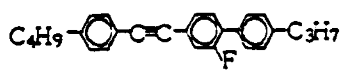 | 10.0 | | |
| 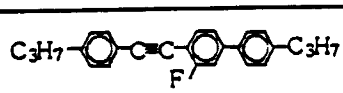 | | 10.0 | |
| 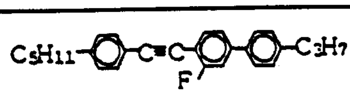 | | 10.0 | |
| 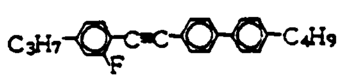 | | | 10.0 |
| 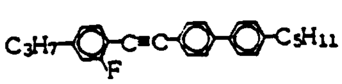 | | | 10.0 |
| 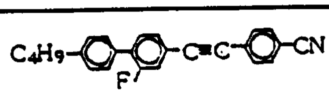 | 5.0 | | |

(proportion: % by weight)

Table 12

| Liquid Crystal Composition | d | e | f | g |
|---|---|---|---|---|
| 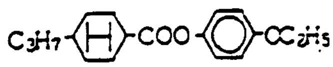 | 6.4 | 5.4 | 4.6 | 5.9 |
| 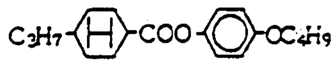 | 16.5 | 13.9 | 12.1 | 15.3 |
| 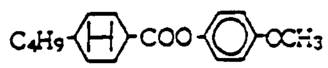 | 12.9 | 10.9 | 9.4 | 11.9 |
| 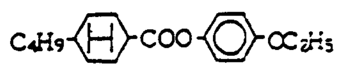 | 13.0 | 10.9 | 9.5 | 11.9 |
| 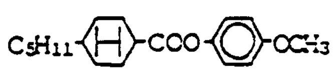 | 14.2 | 11.9 | 10.4 | 13.1 |
| 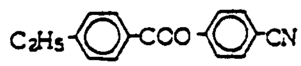 | 14.0 | 12.0 | 12.0 | 6.0 |
| 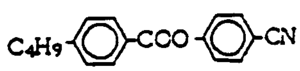 | | 6.0 | 12.0 | 6.0 |
| 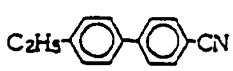 | | 6.0 | | |
| 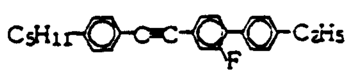 | 9.0 | | | |
| 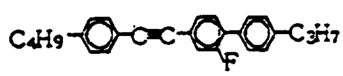 | | | 10.0 | 10.0 |
| 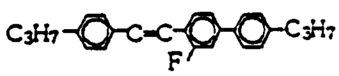 | | | 10.0 | 10.0 |
| 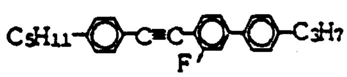 | 9.0 | 9.0 | 10.0 | 10.0 |
| 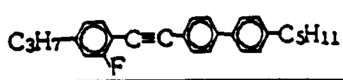 | | 9.0 | | |
| 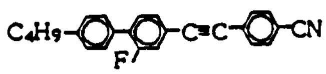 | 5.0 | | | |
| 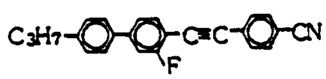 | | 5.0 | | |

(proportion: % by weight)

**Table 13**

| Liquid Crystal Composition | h | i | j |
|---|---|---|---|
| 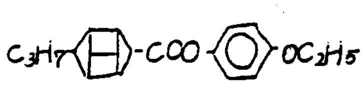 | 5.5 | 5.5 | 4.6 |
| 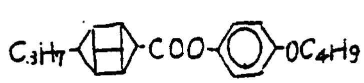 | 14.5 | 14.5 | 11.8 |
| 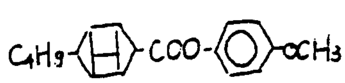 | 11.3 | 11.3 | 9.2 |
| 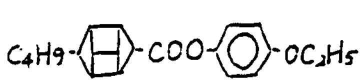 | 11.3 | 11.3 | 9.3 |
| 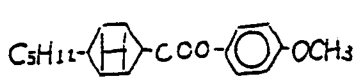 | 12.4 | 12.4 | 10.1 |
| 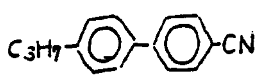 | 5.0 | 5.0 | 5.0 |
| 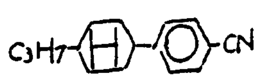 | 5.0 | 10.0 | 10.0 |
| 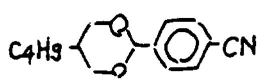 | 15.0 | 10.0 | 10.0 |
| 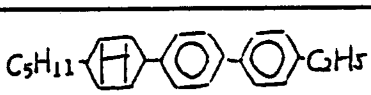 | 10.0 | | |
| 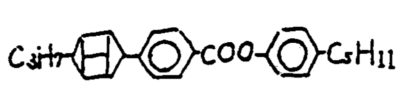 | | 10.0 | 15.0 |
| 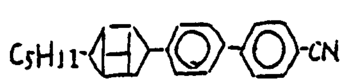 | 10.0 | | 15.0 |
| 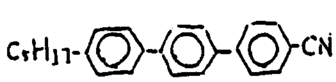 | | 10.0 | |

(proportion: % by weight)

N-I point and $\Delta n$ of each of compositions (a) to (j) were measured and the data obtained are shown below.

Table 14

| Liquid Crystal Composition | a | b | c | d | e |
|---|---|---|---|---|---|
| N-I point (°C) | 63.4 | 76.6 | 75.2 | 82.4 | 80.9 |
| Δn | 0.158 | 0.168 | 0.164 | 0.159 | 0.175 |
| Liquid Crystal Composition | f | g | h | i | j |
| N-I point (°C) | 86.5 | 91.7 | 79.2 | 80.1 | 92.6 |
| Δn | 0.189 | 0.182 | 0.119 | 0.127 | 0.128 |

From the above data, it is understood that liquid crystal compositions (a) to (g) containing compounds of the present invention have a higher $\Delta n$ value by 0.04 to 0.06 than liquid crystal compositions (h) to (j) comprising only known liquid crystal compounds.

Example 8:

Liquid Crystal Display Device

Fig. 1 is referred to. An electrode 3 of a transparent conductive coating (e.g., ITO coating) was formed on each of glass substrates 1 and 2; and an aligning coating of polyimide or the like was coated over the electrode. Next, this was rubbed to form an aligning coating layer 4. The glass substrates 1 and 2 were then positioned to face to each other via a sealant 6, and one of liquid crystal compositions (a) to (j) as prepared in Example 7 was injected into the space between the glass substrates. A polarizing plate 5 was attached to the outer surface of the substrate 1 and a reflective polarizing plate 5 to the outer surface of the substrate 2. Thus, TN type liquid crystal display cells A to J were formed. The cell gap d in each liquid crystal display cell was determined in such a way that the value of $\Delta n \cdot d$ of each cell falls within the range of from 1.2 to 1.3.

The thus formed liquid crystal display cells were driven by an alternating current static driving system, and the threshold voltage at 25°C (hereinafter referred to as Vth), the visible angle dependence of voltage-transmittance (hereinafter referred to as $\alpha$), the sharpness (hereinafter referred to as $\beta$), the rise time (hereinafter referred to as Tr) and the descending speed (hereinafter referred to as Td) were measured by reflection measurement. $\alpha$, $\beta$ and Vth are defined by the following expressions.

$$\alpha = \frac{V_{10}\ (\Theta = 80°\ T = 25°C)}{V_{10}\ (\Theta = 50°\ T = 25°C)}$$

$$\beta = \frac{V_{10}\ (\Theta = 80°\ T = 25°C)}{V_{90}\ (\Theta = 50°\ T = 25°C)}$$

$$Vth = V_{10}$$

$\Theta$: angle of incident rays to cell (the vertical direction to panel is 90°).

$V_{10}$, $V_{90}$: voltage at transmittance of 10 % and 90 %, respectively.

Tr represents the time necessary for lowering the transmittance to 10 % when the voltage was on (the voltage applied was $V_{90}$ ($\Theta$=80°)); and Td represents the time necessary for recovering the transmittance of 90% when the voltage was off (the voltage applied was $V_{10}$ ($\Theta$=80°)). The results of measurement are shown below.

Table 15

| Liquid Crystal Panel | A | B | C | D | E |
|---|---|---|---|---|---|
| Liquid Crystal Composition | a | b | c | d | e |
| Cell Thickness (μ) | 8.0 | 8.0 | 8.0 | 8.0 | 7.0 |
| Δnd | 1.26 | 1.34 | 1.31 | 1.27 | 1.23 |
| $V_{th}$ (V) | 1.214 | 1.747 | 1.664 | 2.005 | 1.530 |
| α | 1.193 | 1.158 | 1.170 | 1.161 | 1.190 |
| β | 1.270 | 1.256 | 1.255 | 1.248 | 1.254 |
| Tr (ms) | 114 | 95 | 95 | 100 | 77 |
| Td (ms) | 150 | 131 | 125 | 139 | 110 |
| Tr + Td (ms) | 264 | 226 | 220 | 239 | 187 |
| Liquid Crystal Panel | F | G | H | I | J |
| Liquid Crystal Composition | f | g | h | i | j |
| Cell Thickness (μ) | 7.0 | 7.0 | 10.0 | 10.0 | 10.0 |
| Δnd | 1.32 | 1.27 | 1.19 | 1.27 | 1.28 |
| $V_{th}$ (V) | 1.635 | 2.396 | 1.645 | 1.667 | 1.719 |
| α | 1.170 | 1.140 | 1.170 | 1.169 | 1.186 |
| β | 1.252 | 1.238 | 1.259 | 1.256 | 1.269 |
| Tr (ms) | 85 | 88 | 128 | 139 | 141 |
| Td (ms) | 108 | 109 | 189 | 191 | 196 |
| Tr + Td (ms) | 193 | 197 | 317 | 330 | 337 |

From the above, it is understood that the liquid crystal cells A to G each having the liquid crystal composition containing liquid crystal compounds of the present invention have a higher (Tr+Td) value by 50 to 150 ms or so than the comparative liquid crystal cells H to J each comprising known liquid crystal compounds only.

In the present example, a TN type liquid crystal display cell was used, but the same results were obtained when an STN type display cell was used.

As mentioned above, the compounds of the present invention are well compatible with other liquid crystal compounds. By blending them with ordinary known liquid crystal compounds, the resulting mixture may effectively have a broadened practical temperature range and an enlarged Δn value. In these points, the compounds of the present invention are effective as base components in liquid crystal compositions for an STN display system which is the main current of display devices in these days.

## Claims

1. A tolan derivative of a general formula:

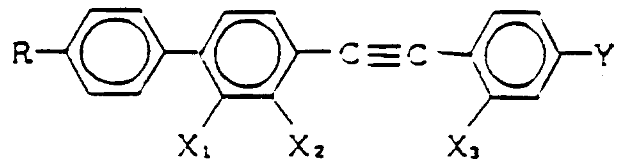

(1)

where R represents a linear alkyl group having from 1 to 10 carbon atoms; X1, X2 and X3 each represent a fluorine atom or a hydrogen atom, provided that one of them is necessarily a fluorine atom, but X1 and X2 are not at the same time fluorine atoms, and the others are hydrogen atoms; and Y represents a nitrile group or a linear alkyl group having form 1 to 10 carbon atoms.

2. The tolan derivative as claimed in claim 1, which is represented by a general formula:

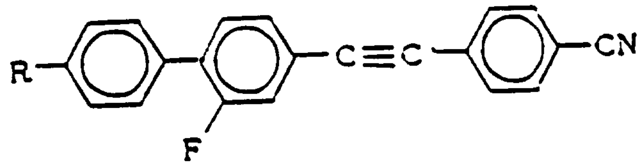

(1-a)

where R represents a linear alkyl group having from 1 to 10 carbon atoms.

3. The tolan derivative as claimed in claim 1, which is represented by a general formula:

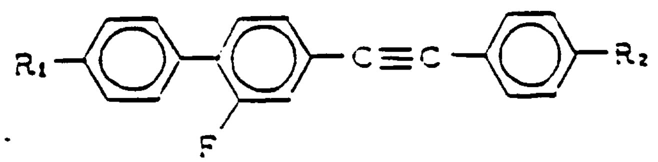

(1-b)

where R1 and R2 each represents a linear alkyl group having from 1 to 10 carbon atoms.

4. The tolan derivative as claimed in claim 1, which is represented by a general formula:

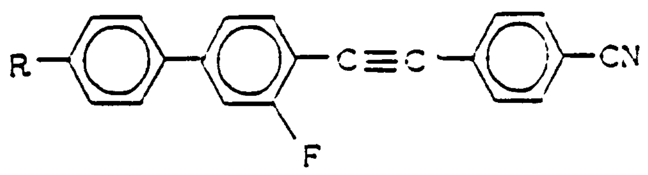

(1-c)

where R represents a linear alkyl group having from 1 to 10 carbon atoms.

5. The tolan derivative as claimed in claim 1, which is represented by a general formula:

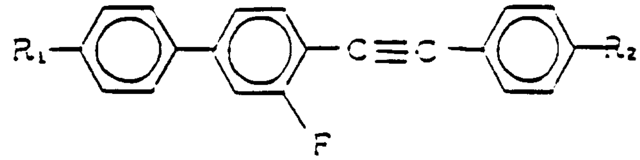

(1-d)

where R1 and R2 each represents a linear alkyl group having from 1 to 10 carbon atoms.

6. The tolan derivative as claimed in claim 1, which is represented by a general formula:

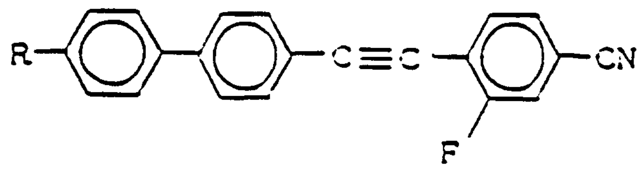

(1-e)

where R represents a linear alkyl group having from 1 to 10 carbon atoms.

7. The tolan derivative as claimed in claim 1, which is represented by a general formula:

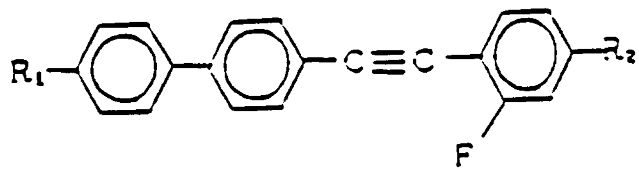

(1-f)

where R1 and R2 each represents a linear alkyl group having from 1 to 10 carbon atoms.

8. A liquid crystal composition containing at least one tolan derivative of a general formula:

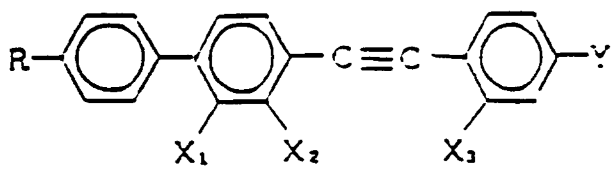

(1)

where R represents a linear alkyl group having from 1 to 10 carbon atoms; X1, X2 and X3 each represent a fluorine atom or a hydrogen atom, provided that one of them is necessarily a fluorine atom, but X1 and X2 are not at the same time fluorine atoms, and the others are hydrogen atoms; and Y represents a nitrile group or a linear alkyl group having form 1 to 10 carbon atoms.

9. The liquid crystal composition as claimed in claim 8, which contains from 5 to 50 % by weight of at least one tolan derivative of a general formula:

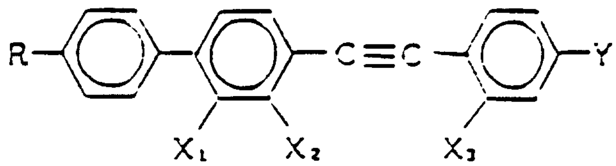

(1)

where R represents a linear alkyl group having from 1 to 10 carbon atoms; X1, X2 and X3 each represent a fluorine atom or a hydrogen atom, provided that one of them is necessarily a fluorine atom, but X1 and X2 are not at the same time fluorine atoms, and the others are hydrogen atoms; and Y represents a nitrile group or a linear alkyl group having form 1 to 10 carbon atoms; and

from 30 to 80 % by weight of at least one phenyl cyclohexanecarboxylate derivative (hereinafter referred to as ECH derivative) of a general formula:

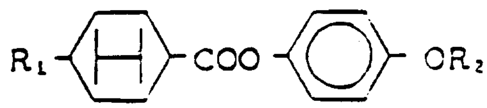

where R1 and R2 each represent a linear alkyl group having from 1 to 10 carbon atoms; and the cyclohexane ring is in the trans-configuration in the formula.

**10.** The liquid crystal composition as claimed in claim 8, which contains from 10 to 35 % by weight of at least one tolan derivative of a general formula:

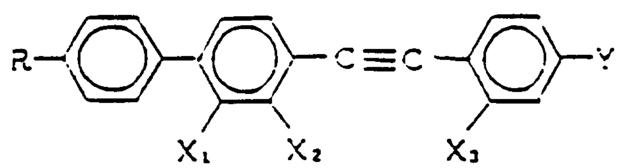

(1)

where R represents a linear alkyl group having from 1 to 10 carbon atoms; X1, X2 and X3 each represent a fluorine atom or a hydrogen atom, provided that one of them is necessarily a fluorine atom, but X1 and X2 are not at the same time fluorine atoms, and the others are hydrogen atoms; and Y represents a nitrile group or a linear alkyl group having form 1 to 10 carbon atoms; and

from 40 to 70 % by weight of at least one ECH derivative of a general formula:

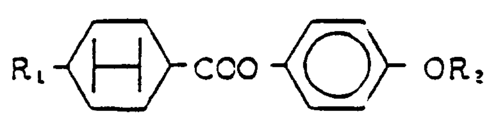

where R1 and R2 each represent a linear alkyl group having from 1 to 10 carbon atoms; and the cyclohexane ring is in the trans-configuration in the formula; and

from 10 to 50 % by weight of at least one compound having positive dielectric anisotropy of a general formula:

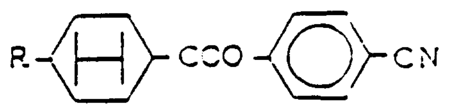

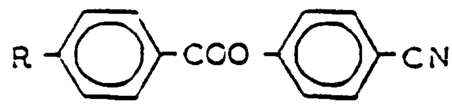

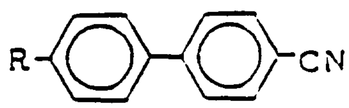

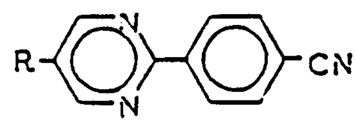

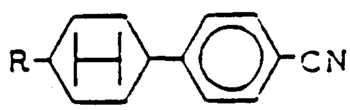

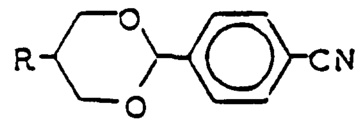

where R represents a linear alkyl group having from 1 to 10 carbon atoms; and the cyclohexane ring is in the transconfiguration in the formula.

**11.** A liquid crystal display device having a liquid crystal composition containing at least one tolan derivative of a general formula:

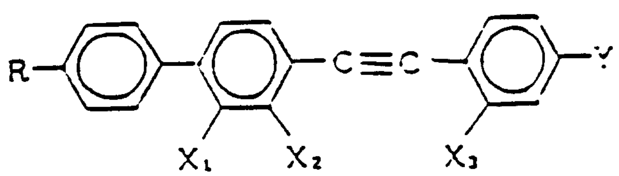

(1)

where R represents a linear alkyl group having from 1 to 10 carbon atoms; X1, X2 and X3 each represent a fluorine atom or a hydrogen atom, provided that one of them is necessarily a fluorine atom, but X1 and X2 are not at the same time fluorine atoms, and the others are hydrogen atoms; and Y represents a nitrile group or a linear alkyl group having form 1 to 10 carbon atoms.

**Patentansprüche**

**1.** Tolanderivat der allgemeinen Formel

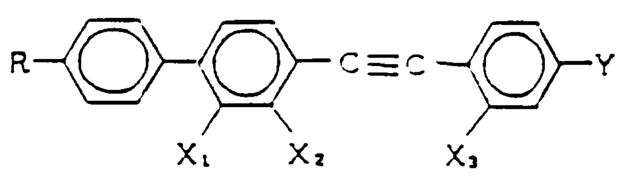

(1)

worin R eine lineare Alkylgruppe mit 1 bis 10 Kohlenstoffatomen bedeutet; $X_1$, $X_2$ und $X_3$ jeweils ein Fluoratom oder ein Wasserstoffatom bedeuten, mit der Maßgabe, daß einer dieser Reste notwendigerweise ein Fluoratom bedeutet, jedoch $X_1$ und $X_2$ nicht gleichzeitig Fluoratome bedeuten, und die übrigen Reste Wasserstoffatome sind; und Y eine Nitrilgruppe oder eine lineare Alkylgruppe mit 1 bis 10 Kohlenstoffatomen bedeutet.

2. Tolanderivat nach Anspruch 1 der allgemeinen Formel

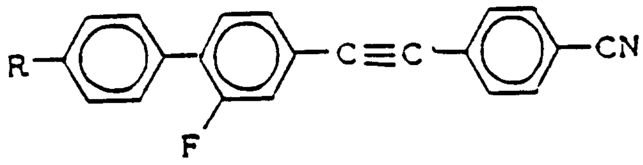

(1-a)

worin R eine lineare Alkylgruppe mit 1 bis 10 Kohlenstoffatomen bedeutet.

3. Tolanderivat nach Anspruch 1 der allgemeinen Formel

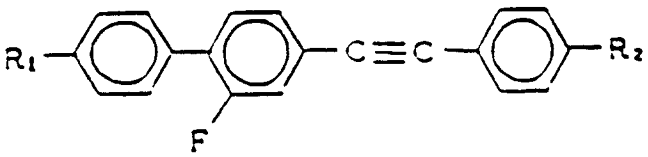

(1-b)

worin $R_1$ und $R_2$ jeweils eine lineare Alkylgruppe mit 1 bis 10 Kohlenstoffatomen bedeuten.

4. Tolanderivat nach Anspruch 1 der allgemeinen Formel

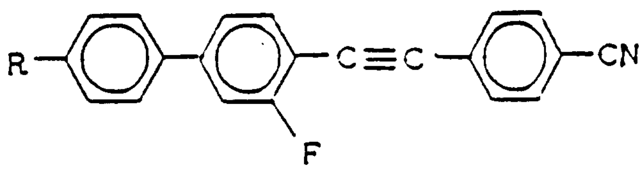

(1-c)

worin R eine lineare Alkylgruppe mit 1 bis 10 Kohlenstoffatomen bedeutet.

5. Tolanderivat nach Anspruch 1 der allgemeinen Formel

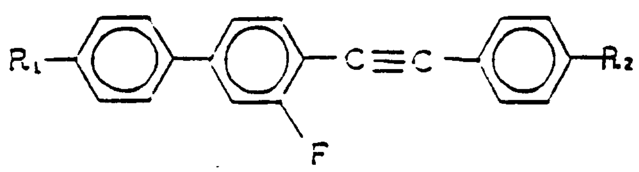

(1-d)

worin $R_1$ und $R_2$ jeweils lineare Alkylgruppen mit 1 bis 10 Kohlenstoffatomen bedeuten.

6. Tolanderivat nach Anspruch 1 der allgemeinen Formel

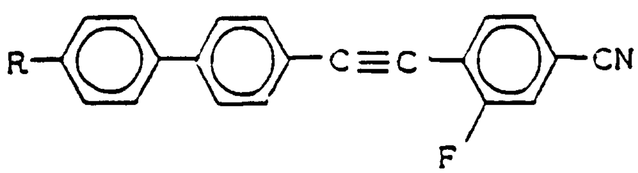

(1-e)

worin R eine lineare Alkylgruppe mit 1 bis 10 Kohlenstoffatomen bedeutet.

**7.** Tolanderivat nach Anspruch 1 der allgemeinen Formel

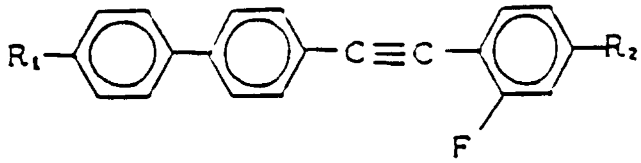

(I-f)

worin $R_1$ und $R_2$ jeweils eine lineare Alkylgruppe mit 1 bis 10 Kohlenstoffatomen bedeuten.

**8.** Flüssigkristall-Zusammensetzung mit einem Gehalt an mindestens einem Tolanderivat der allgemeinen Formel

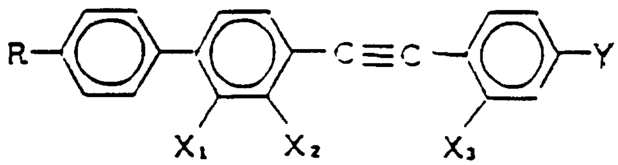

(I)

worin R eine lineare Alkylgruppe mit 1 bis 10 Kohlenstoffatomen bedeutet; $X_1$, $X_2$ und $X_3$ jeweils ein Fluoratom oder ein Wasserstoffatom bedeuten, mit der Maßgabe, daß einer dieser Reste notwendigerweise ein Fluoratom bedeutet, jedoch $X_1$ und $X_2$ nicht gleichzeitig Fluoratome bedeuten, und die übrigen Reste Wasserstoffatome sind; und Y eine Nitrilgruppe oder eine lineare Alkylgruppe mit 1 bis 10 Kohlenstoffatomen bedeutet.

**9.** Flüssigkristall-Zusammensetzung nach Anspruch 8, enthaltend 5 bis 50 Gew.-% mindestens eines Tolanderivats der allgemeinen Formel

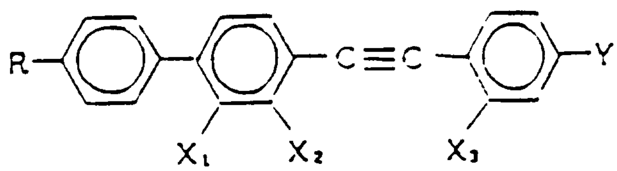

(I)

worin R eine lineare Alkylgruppe mit 1 bis 10 Kohlenstoffatomen bedeutet; $X_1$, $X_2$ und $X_3$ jeweils ein Fluoratom oder ein Wasserstoffatom bedeuten, mit der Maßgabe, daß einer dieser Reste notwendigerweise ein Fluoratom bedeutet, jedoch $X_1$ und $X_2$ nicht gleichzeitig Fluoratome bedeuten, und die übrigen Reste Wasserstoffatome sind; und Y eine Nitrilgruppe oder eine lineare Alkylgruppe mit 1 bis 10 Kohlenstoffatomen bedeutet; und

30 bis 80 Gew.-% mindestens eines Phenylcyclohexancarboxylatderivats (nachstehend als ECH-Derivat bezeichnet) der allgemeinen Formel

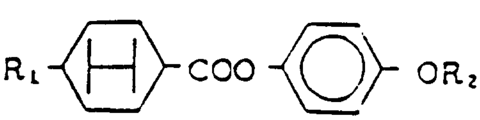

worin $R_1$ und $R_2$ jeweils eine lineare Alkylgruppe mit 1 bis 10 Kohlenstoffatomen bedeuten und der Cyclohexanring in der Formel in der trans-Konfiguration vorliegt.

**10.** Flüssigkristall-Zusammensetzung nach Anspruch 8, enthaltend 10 bis 35 Gew.-% mindestens eines Tolanderivats der allgemeinen Formel

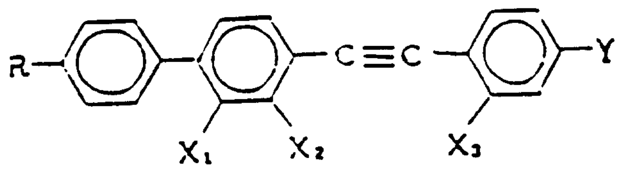

(1)

worin R eine lineare Alkylgruppe mit 1 bis 10 Kohlenstoffatomen bedeutet; $X_1$, $X_2$ und $X_3$ jeweils ein Fluoratom oder ein Wasserstoffatom bedeuten, mit der Maßgabe, daß einer dieser Reste notwendigerweise ein Fluoratom bedeutet, jedoch $X_1$ und $X_2$ nicht gleichzeitig Fluoratome bedeuten, und die übrigen Reste Wasserstoffatome sind; und Y eine Nitrilgruppe oder eine lineare Alkylgruppe mit 1 bis 10 Kohlenstoffatomen bedeutet; und

40 bis 70 Gew.-% mindestens eines ECH-Derivats der allgemeinen Formel

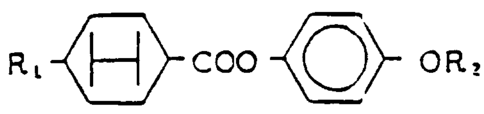

worin $R_1$ und $R_2$ jeweils eine lineare Alkylgruppe mit 1 bis 10 Kohlenstoffatomen bedeuten und der Cyclohexanring in der Formel in der trans-Konfiguration vorliegt; und

10 bis 50 Gew.-% mindestens einer Verbindung mit einer positiven dielektrischen Anisotropie der allgemeinen Formel

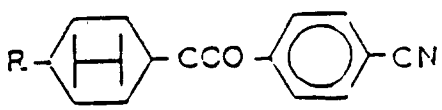

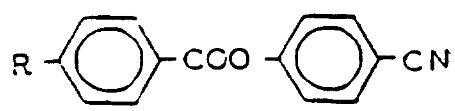

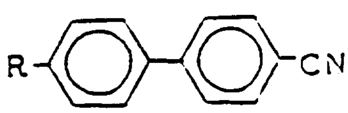

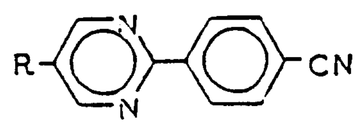

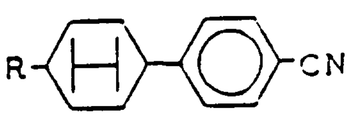

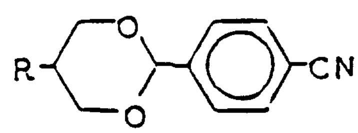

worin R eine lineare Alkylgruppe mit 1 bis 10 Kohlenstoffatomen bedeutet und der Cyclohexanring in der Formel in der trans-Konfiguration vorliegt.

11. Flussigkristall-Anzeigevorrichtung, enthaltend mindestens ein Tolanderivat der allgemeinen Formel

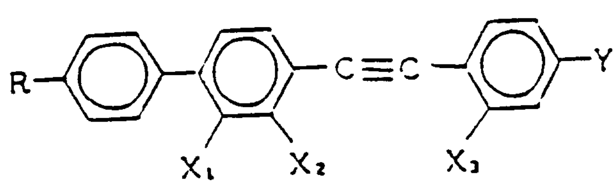

(1)

worin R eine lineare Alkylgruppe mit 1 bis 10 Kohlenstoffatomen bedeutet; $X_1$, $X_2$ und $X_3$ jeweils ein Fluoratom oder ein Wasserstoffatom bedeuten, mit der Maßgabe, daß einer dieser Reste notwendigerweise ein Fluor-

atom bedeutet, jedoch $X_1$ und $X_2$ nicht gleichzeitig Fluoratome bedeuten, und die übrigen Reste Wasserstoffatome sind; und Y eine Nitrilgruppe oder eine lineare Alkylgruppe mit 1 bis 10 Kohlenstoffatomen bedeutet.

**Revendications**

1. Dérivé de tolane de formule générale :

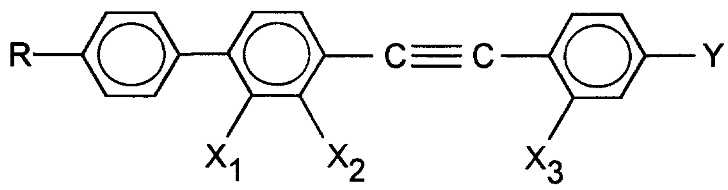

(1)

dans laquelle R représente un groupe alkyle linéaire possédant de 1 à 10 atomes de carbone; $X_1$, $X_2$ et $X_3$ représentent chacun un atome de fluor ou un atome d'hydrogène, avec la condition que l'un d'entre eux soit nécessairement un atome de fluor, mais que $X_1$ et $X_2$ ne soient pas en même temps des atomes de fluor, et les autres sont des atomes d'hydrogène; et Y représente un groupe nitrile ou un groupe alkyle linéaire possédant de 1 à 10 atomes de carbone.

2. Dérivé de tolane selon la revendication 1, représenté par la formule générale :

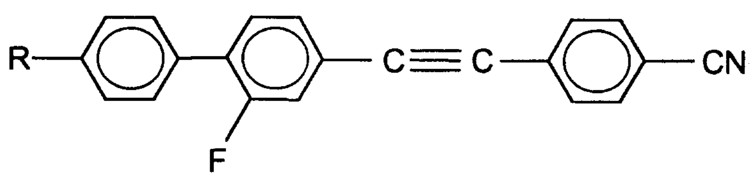

(1-a)

dans laquelle R représente un groupe alkyle linéaire possédant de 1 à 10 atomes de carbone.

3. Dérivé de tolane selon la revendication 1, représenté par la formule générale :

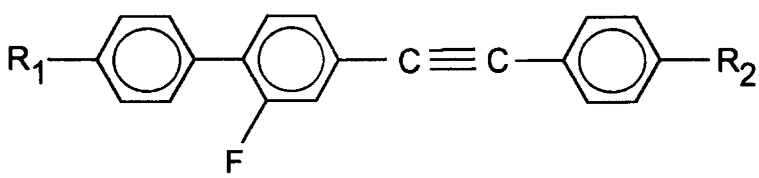

(1-b)

dans laquelle $R_1$ et $R_2$ représentent chacun un groupe alkyle linéaire possédant de 1 à 10 atomes de carbone.

4. Dérivé de tolane selon la revendication 1, représenté par la formule générale :

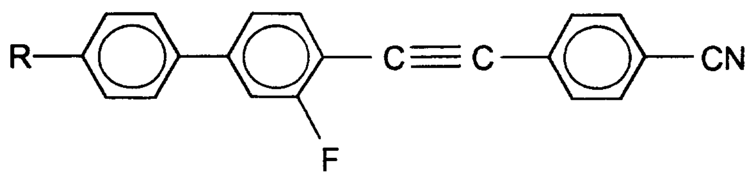

(1-c)

dans laquelle R représente un groupe alkyle linéaire possédant de 1 à 10 atomes de carbone.

**5.** Dérivé de tolane selon la revendication 1, représenté par la formule générale :

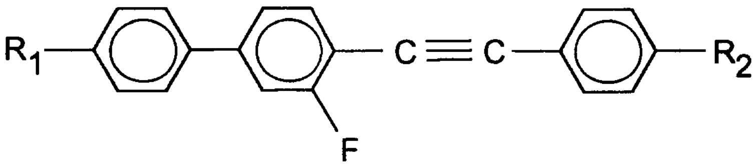

(1-d)

dans laquelle $R_1$ et $R_2$ représentent chacun un groupe alkyle linéaire possédant de 1 à 10 atomes de carbone.

**6.** Dérivé de tolane selon la revendication 1, représenté par la formule générale :

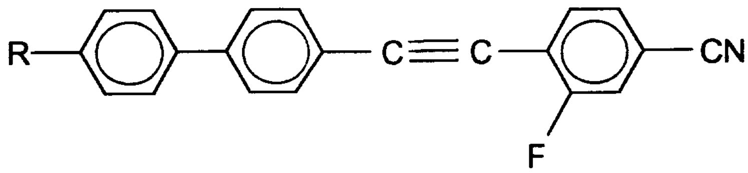

(1-e)

dans laquelle R représente un groupe alkyle linéaire possédant de 1 à 10 atomes de carbone.

**7.** Dérivé de tolane selon la revendication 1, représenté par la formule générale :

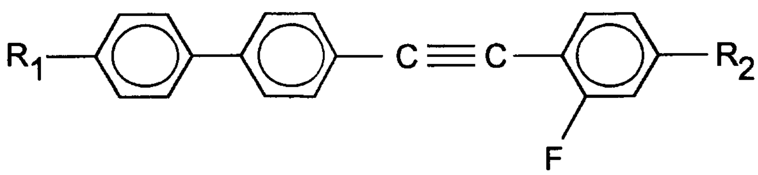

(1-f)

dans laquelle $R_1$ et $R_2$ représentent chacun un groupe alkyle linéaire possédant de 1 à 10 atomes de carbone.

**8.** Composition de cristaux liquides contenant au moins un dérivé de tolane de formule générale :

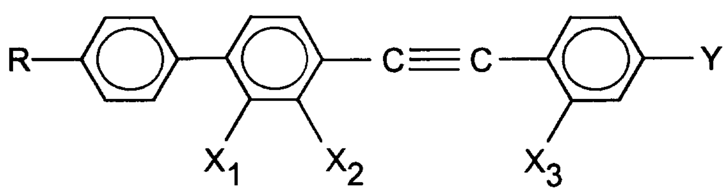

(1)

dans laquelle R représente un groupe alkyle linéaire possédant de 1 à 10 atomes de carbone; $X_1$, $X_2$ et $X_3$ représentent chacun un atome de fluor ou un atome d'hydrogène, avec la condition que l'un d'entre eux soit nécessairement un atome de fluor, mais que $X_1$ et $X_2$ ne soient pas en même temps des atomes de fluor, et les autres sont des atomes d'hydrogène; et Y représente un groupe nitrile ou un groupe alkyle linéaire possédant de 1 à 10 atomes de carbone.

**9.** Composition de cristaux liquides selon la revendication 8, contenant de 5 à 50 % en poids d'au moins un dérivé de tolane de formule générale :

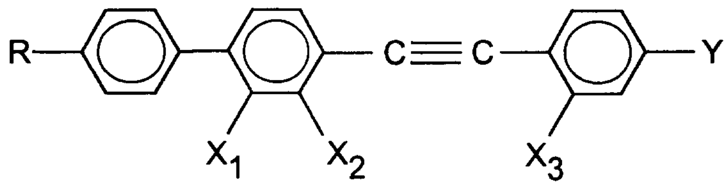

(1)

dans laquelle R représente un groupe alkyle linéaire possédant de 1 à 10 atomes de carbone; $X_1$, $X_2$ et $X_3$ représentent chacun un atome de fluor ou un atome d'hydrogène, avec la condition que l'un d'entre eux soit nécessairement un atome de fluor, mais que $X_1$ et $X_2$ ne soient pas en même temps des atomes de fluor, et les autres sont des atomes d'hydrogène; et Y représente un groupe nitrile ou un groupe alkyle linéaire possédant de 1 à 10 atomes de carbone; et

de 30 à 80 % en poids d'au moins un dérivé de phénylcyclohexanecarboxylate (qui sera désigné par la suite par dérivé ECH) de formule générale :

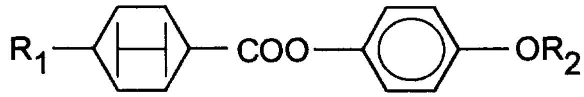

dans laquelle $R_1$ et $R_2$ représentent chacun un groupe alkyle linéaire possédant de 1 à 10 atomes de carbone; et l'anneau de cyclohexane est dans la configuration trans dans la formule.

**10.** Composition de cristaux liquides selon la revendication 8, contenant de 10 à 35 % en poids d'au moins un dérivé de tolane de formule générale :

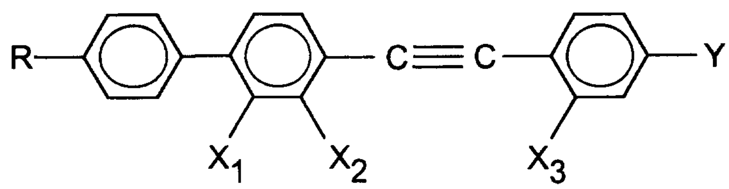

(1)

dans laquelle R représente un groupe alkyle linéaire possédant de 1 à 10 atomes de carbone; $X_1$, $X_2$ et $X_3$ représentent chacun un atome de fluor ou un atome d'hydrogène, avec la condition que l'un d'entre eux soit nécessairement un atome de fluor, mais que $X_1$ et $X_2$ ne soient pas en même temps des atomes de fluor, et les autres sont des atomes d'hydrogène; et Y représente un groupe nitrile ou un groupe alkyle linéaire possédant de 1 à 10 atomes de carbone; et

de 40 à 70 % en poids d'au moins un dérivé ECH de formule générale :

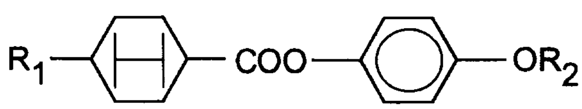

dans laquelle $R_1$ et $R_2$ représentent chacun un groupe alkyle linéaire possédant de 1 à 10 atomes de carbone; et l'anneau de cyclohexane est dans la configuration trans dans la formule; et

de 10 à 50 % en poids d'au moins un composé ayant une anisotropie diélectrique positive de formule générale :

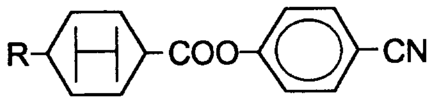 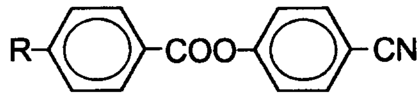

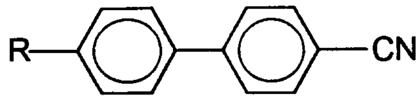 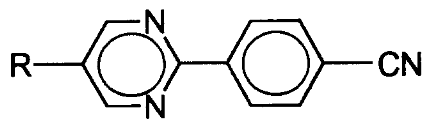

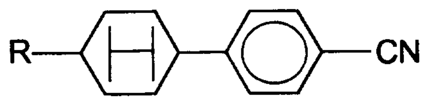 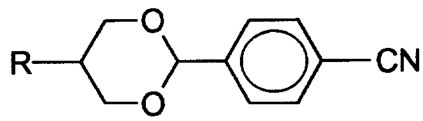

dans laquelle R représente un groupe alkyle linéaire possédant de 1 à 10 atomes de carbone; et l'anneau de cyclohexane est dans la configuration trans dans la formule.

11. Dispositif d'affichage à cristaux liquides comprenant une composition de cristaux liquides contenant au moins un dérivé de tolane de formule générale :

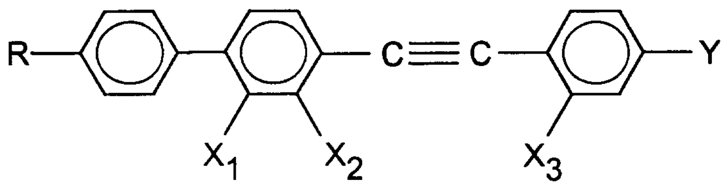 (1)

dans laquelle R représente un groupe alkyle linéaire possédant de 1 à 10 atomes de carbone; $X_1$, $X_2$ et $X_3$ représentent chacun un atome de fluor ou un atome d'hydrogène, avec la condition que l'un d'eux soit nécessairement un atome de fluor, mais que $X_1$ et $X_2$ ne soient pas en même temps des atomes de fluor, et les autres sont des atomes d'hydrogène; et Y représente un groupe nitrile ou un groupe alkyle linéaire possédant de 1 à 10 atomes de carbone.

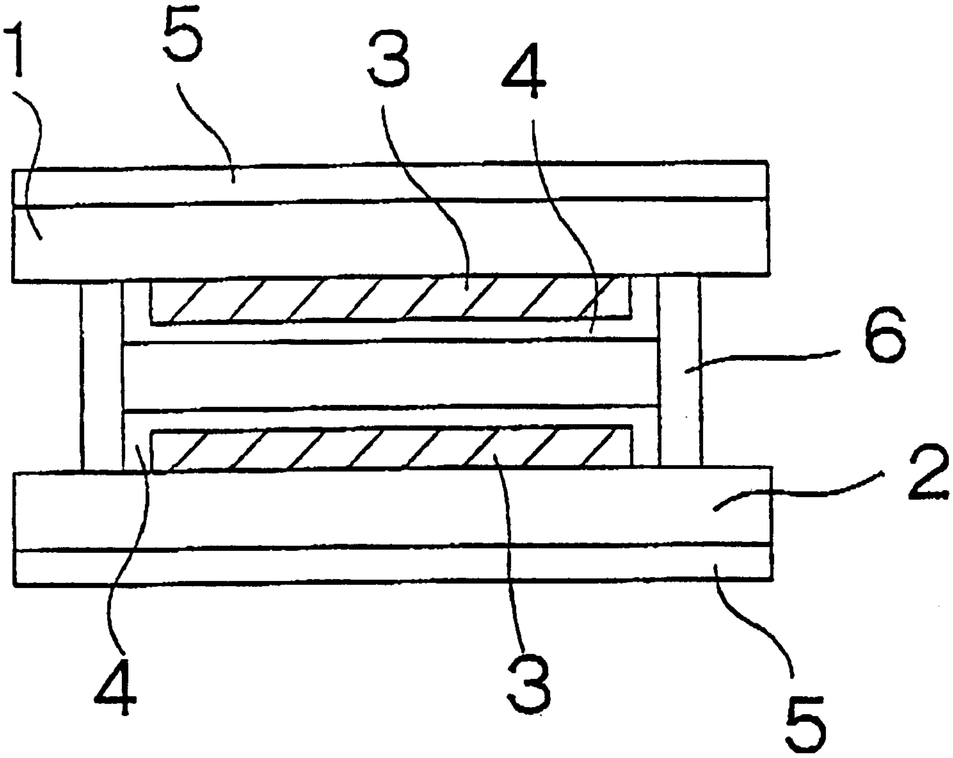


FIG. 1